# EUROPEAN PATENT SPECIFICATION

(11) **EP 1 578 353 B1**
(45) Date of publication and mention of the grant of the patent: **23.03.2011**
(21) Application number: 03726938.8
(22) Date of filing: 19.05.2003
(51) Int. Cl.: A61K 31/4545, A61P 31/06, C07D 211/46

(54) **4-hydroxypiperidine derivatives useful as antimycobacterial agents**
4-HYDROXYPIPERIDIN-DERIVATE ZUR VERWENDUNG ALS ANTIMYCOBAKTERIELLE MITTEL
DÉRIVÉS DE 4-HYDROXYPIPÉRIDINE EN TANT QU'AGENTS ANTIMYCOBACTÉRIENS

(30) Priority: 17.05.2002 US 381244 P; 17.05.2002 US 147587
(43) Date of publication of application: 28.09.2005
(73) Proprietor: Sequella, Inc., Rockville, MD 20850 (US)
(72) Inventor: PROTOPOPOVA, Marina, Nikolaevna, Silver Spring, MD 30910 (US); BOGATCHEVA, Elena, Bethesda, MD 20816 (US)
(74) Representative: Stevens, Ian Edward
(86) International application number: PCT/US2003/015925
(87) International publication number: WO 2003/096987

(56) References cited:
- SHEPHERD, R.G. ET AL.: "Structure-activity studies leading to ethambutol, a new type of antituberculous compound." ANN. N. Y. ACAD. SCI., vol. 135, 1966, pages 686-710, XP002569956

## Description

### FIELD OF INVENTION

The present invention relates to compounds and compositions for treating disease caused bv microorganisms, particularly tuberculosis. The present invention also relates to compounds compounds and compositions having improved anti-mycobacterial activity.

### BACKGROUND OF THE INVENTION

Mycobacterial infections often manifest as diseases such as tuberculosis. Human infections caused by mycobacteria have been widespread since ancient times, and tuberculosis remains a leading cause of death today. Although the incidence of the disease declined, in parallel with advancing standards of living, since the mid-nineteenth century, mycobacterial diseases still constitute a leading cause of morbidity and mortality in countries with limited medical resources. Additionally, mycobacterial diseases can cause overwhelming, disseminated disease in immunocompromised patients. In spite of the efforts of numerous health organizations worldwide, the eradication of mycobacterial diseases has never been achieved, nor is eradication imminent. Nearly one third of the world's population is infected with *mycobacterium tuberculosis* complex, commonly referred to as tuberculosis (TB), with approximately 8 million new cases, and two to three million deaths attributable to TB yearly. Tuberculosis (TB) is the cause of the largest number of human deaths attributable to a single etiologic agent (see Dye et al., J. Am. Med. Association, 282, 677-686, (1999); and 2000 WHO/OMS Press Release).

After decades of decline, TB is now on the rise. In the United States, up to 10 million individuals are believed to be infected. Almost 28,000 new cases were reported in 1990, constituting a 9.4 percent increase over 1989. A sixteen percent increase in TB cases was observed from 1985 to 1990. Overcrowded living conditions and shared air spaces are especially conducive to the spread of TB, contributing to the increase in instances that have been observed among prison inmates, and among the homeless in larger U.S. cities. Approximately half of all patients with "Acquired Immune Deficiency Syndrome" (AIDS) will acquire a mycobacterial infection, with TB being an especially devastating complication. AIDS patients are at higher risks of developing clinical TB, and anti-TB treatment seems to be less effective than in non-AIDS patients. Consequently, the infection often progresses to a fatal disseminated disease.

Mycobacteria other than *M. tuberculosis* are increasingly found in opportunistic infections that plague the AIDS patient. Organisms from the *M. avium-intracellulare* complex (MAC), especially serotypes four and eight, account for 68% of the mycobacterial isolates from AIDS patients. Enormous numbers of MAC are found (up to 10¹⁰ acid-fast bacilli per gram of tissue), and consequently, the prognosis for the infected AIDS patient is poor.

The World Health Organization (WHO) continues to encourage the battle against TB, recommending prevention initiatives such as the "Expanded Program on Immunization" (EPI), and therapeutic compliance initiatives such as "Directly Observed Treatment Short-Course" (DOTS). For the eradication of TB, diagnosis, treatment, and prevention are equally important. Rapid detection of active TB patients will lead to early treatment by which about 90% cure is expected. Therefore, early diagnosis is critical for the battle against TB. In addition, therapeutic compliance will ensure not only elimination of infection, but also reduction in the emergence of drug-resistance strains.

The emergence of drug-resistant *M. tuberculosis* is an extremely disturbing phenomenon. The rate of new TB cases proven resistant to at least one standard drug increased from 10 percent in the early 1980's to 23 percent in 1991. Compliance with therapeutic regimens, therefore, is also a crucial component in efforts to eliminate TB and prevent the emergence of drug resistant strains. Equally important is the development of new therapeutic agents that are effective as vaccines, and as treatments, for disease caused by drug resistant strains of mycobacteria.

Although over 37 species of mycobacteria have been identified, more than 95% of all human infections are caused by six species of mycobacteria: *M. tuberculosis, M. avium intracellulare, M. kansasii, M. fortuitum, M. chelonae,* and *M. leprae.* The most prevalent mycobacterial disease in humans is tuberculosis (TB) which is predominantly caused by mycobacterial species comprising *M. tuberculosis, M. bovis,* or *M. africanum* (Merck Manual 1992). Infection is typically initiated by the inhalation of infectious particles which are able to reach the terminal pathways in lungs. Following engulfment by alveolar macrophages, the bacilli are able to replicate freely, with eventual destruction of the phagocytic cells. A cascade effect ensues wherein destruction of the phagocytic cells causes additional macrophages and lymphocytes to migrate to the site of infection, where they too are ultimately eliminated. The disease is further disseminated during the initial stages by the infected macrophages which travel to local lymph nodes, as well as into the blood stream and other tissues such as the bone marrow, spleen, kidneys, bone and central nervous system. (See Murray et al. Medical Microbiology, The C.V. Mosby Company 219-230 (1990)).

There is still no clear understanding of the factors which contribute to the virulence of mycobacteria. Many investigators have implicated lipids of the cell wall and bacterial surface as contributors to colony morphology and virulence. Evidence suggests that C-mycosides, on the surface of certain mycobacterial cells, are important in facilitating survival of the organism within macrophages. Trehalose 6,6' dimycolate, a cord factor, has been implicated for other mycobacteria.

The interrelationship of colony morphology and virulence is particularly pronounced in *M. avium. M. avium* bacilli occur in several distinct colony forms. Bacilli which grow as transparent, or rough, colonies on conventional laboratory media are multiplicable within macrophages in tissue culture, are virulent when injected into susceptible mice, and are resistant to antibiotics. Rough or transparent bacilli, which are maintained on laboratory culture media, often spontaneously assume an opaque R colony morphology, at which time they are not multiplicable in macrophages, are avirulent in mice, and are highly susceptible to antibiotics. The differences in colony morphology between the transparent, rough and opaque strains of *M*. *avium* are almost certainly due to the presence of a glycolipid coating on the surface of transparent and rough organisms which acts as a protective capsule. This capsule, or coating, is composed primarily of C-mycosides which apparently shield the virulent *M. avium* organisms from lysosomal enzymes and antibiotics. By contrast, the non-virulent opaque forms of *M. avium* have very little C-mycoside on their surface. Both the resistance to antibiotics and the resistance to killing by macrophages have been attributed to the glycolipid barrier on the surface of *M. avium.*

Diagnosis of mycobacterial infection is confirmed by the isolation and identification of the pathogen, although conventional diagnosis is based on sputum smears, chest X-ray examination (CXR), and clinical symptoms. Isolation of mycobacteria on a medium takes as long as four to eight weeks. Species identification takes a further two weeks. There are several other techniques for detecting mycobacteria such as the polymerase chain reaction (PCR), mycobacterium tuberculosis direct test, or amplified mycobacterium tuberculosis direct test (MTD), and detection assays that utilize radioactive labels.

One diagnostic test that is widely used for detecting infections caused by *M*. *tuberculosis* is the tuberculin skin test. Although numerous versions of the skin test are available, typically one of two preparations of tuberculin antigens are used: old tuberculin (OT), or purified protein derivative (PPD). The antigen preparation is either injected into the skin intradermally, or is topically applied and is then invasively transported into the skin with the use of a multiprong inoculator (Tine test). Several problems exist with the skin test diagnosis method. For example, the Tine test is not generally recommended because the amount of antigen injected into the intradermal layer cannot be accurately controlled. (See Murray et al. Medical Microbiology, The C.V. Mosby Company 219-230 (1990)).

Although the tuberculin skin tests are widely used, they typically require two to three days to generate results, and many times, the results are inaccurate since false positives are sometimes seen in subjects who have been exposed to mycobacteria, but are healthy. In addition, instances of mis-diagnosis are frequent since a positive result is observed not only in active TB patients, but also in persons vaccinated with Bacille Calmette-Guerin (BCG), and those who had been infected with mycobacteria, but have not developed the disease. It is hard therefore, to distinguish active TB patients from the others, such as household TB contacts, by the tuberculin skin test. Additionally, the tuberculin test often produces a cross-reaction in those individuals who were infected with mycobacteria other than *M. tuberculosis* (MOTT). Therefore, diagnosis using the skin tests currently available is frequently subject to error and inaccuracies.

The standard treatment for tuberculosis caused by drug-sensitive organisms is a six-month regimen consisting of four drugs given for two months, followed by two drugs given for four months. The two most important drugs, given throughout the six-month course of therapy, are isoniazid and rifampin. Although the regimen is relatively simple, its administration is quite complicated. Daily ingestion of eight or nine pills is often required during the first phase of therapy; a daunting and confusing prospect. Even severely ill patients are often symptom free within a few weeks, and nearly all appear to be cured within a few months. If the treatment is not continued to completion, however, the patient may experience a relapse, and the relapse rate for patients who do not continue treatment to completion is high. A variety of forms of patient-centered care are used to promote adherence with therapy. The most effective way of ensuring that patients are taking their medication is to use directly observed therapy, which involves having a member of the health care team observe the patient take each dose of each drug. Directly observed therapy can be provided in the clinic, the patient's residence, or any mutually agreed upon site. Nearly all patients who have tuberculosis caused by drug-sensitive organisms, and who complete therapy will be cured, and the risk of relapse is very low ("Ending Neglect: The Elimination of Tuberculosis in the United States" ed. L. Geiter Committee on the Elimination of Tuberculosis in the United States Division of Health Promotion and Disease Prevention, Institute of Medicine. Unpublished.)

What is needed are effective therapeutic regimens that include improved vaccination and treatment protocols. Currently available therapeutics are no longer consistently effective as a result of the problems with treatment compliance, and these compliance problems contribute to the development of drug resistant mycobacterial strains.

Ethambutol (EMB) is a widely used antibiotic for the treatment of TB, with over 300 million doses delivered for tuberculosis therapy in 1988.

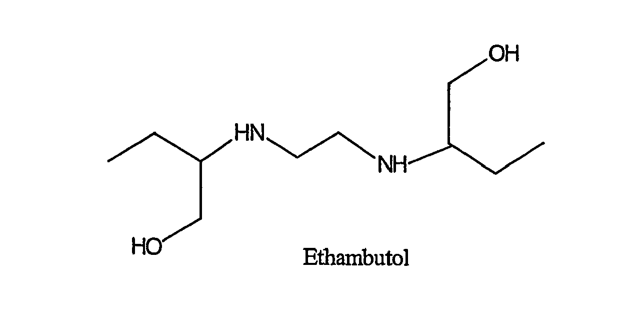

Ethambutol, developed by Lederle Laboratories in the 1950s, has low toxicity and is a good pharmacokinetic. However, ethambutol has a relatively high Minium Inhibition Concentration (MIC) of about 5 µg/ml, and can cause optic neuritis. Thus, there is an increasing need for new, and more effective, therapeutic compositions (See for example, U.S. Pat. No. 3,176,040, U.S. Pat. No. 4,262,122; U.S. Pat No. 4,006,234; U.S. Pat. No. 3,931,157; U.S. Pat. No. 3, 931,152; U.S. Re. 29,358; and Häusler et al., Bioorganic & Medicinal Chemistry Letters 11 (2001) 1679-1681). In the decoder years since the discovery of the beneficial effects of ethambutol, few pharmacological advances in TB treatmeat have been developed. Moreover, with the combined emergence of drug resistant strains, and the more prevalent spread of mycobacterial disease, it is becoming seriously apparent that new therapeutic compositions are crucial in the fight against tuberculosis.

Clearly effective therapeutic regimens that include improved vaccination and treatment protocols are needed. A therapeutic vaccine that would prevent the onset of tuberculosis, and therefore eliminate the need for therapy is desirable. Although currently available therapeutics such as ethambutol are effective, the emergence of drug resistant strains has necessitated new formulations and compositions that are more versatile than ethambutol. Currently available therapeutics are no longer consistently effective as a result of the problems with treatment compliance, lending to the development of drug resistant mycobacterial strains. What is needed are new anti-tubercular drugs that provide highly effective treatment, and shorten or simplify tuberculosis chemotherapy.

### SUMMARY OF THE INVENTION

The present invention relates to the subject matter of appended claims 1 to 16.

An object of the present invention is to provide methods and compositions for the treatment and prevention of mycobacterial disease, including but not limited to, tuberculosis.

Yet another object of the present invention is to provide composition for the therapeutic formulations for the treatment and prevention of mycobacterial disease.

Another object of the present invention is to provide compositions for therapeutic formulations for the treatment and prevention of mycobacterial disease caused by mycobacterial species comprising *M. tuberculoris* complex, *M. avium intracellulare, M. kansarii, M. fortuitum, M. chelonoe, M. leprae, M. africanium, M. microti, M. avium paratuberculosis,* or *M. bovis.*

These and other objects, features and advantages of the present invention will become apparent after a review of the following detailed description of the disclosed embodiments and the appended claims.

### BRIBF DESCRIPTION OF THE FIGURES

Figure 1 provides representative examples of comparative diamine products synthesized from amino alcohol pre-loaded resina.
Figure 2 provides commercially available amino alcohol pre-loaded resins.
Figure 3 provides Table 1 which shows the prepared library of targeted 21,120 ethambutol analogs.
Figure 4 provides Scheme 1, a schematic showing the Synthesis of the original 100,000 compound library of Ethambutol analogs.
Figure 5 provides Scheme 2, a schematic showing the use of amino alcohol pre-loaded resins and amino acids as linker.
Figure 6 provides Scheme 3, a schematic showing further modification of the linker: working with amino alcohol pro-loaded resins.
Figure 7 provides Table 2 which lists the Amino acids used in the library preparation.
Figure 8 provides representative carbonyl compounds used as reagents in the syntheses.
Figure 9 provides representative examples ofMIC and Lux data
Figure 10 shows the occurrence of alkylating monomers in the active molecules.
Figure 11 provides a list of hit compounds and their strucures including comparative compounds and structures.
Figure 12 provides Table 3 which shows the layout for deconvolutions.

### DETAILED DESCRIPTION

The present invention may be understood more readily by reference to the following detailed description of the specific embodiments included herein. However, although the present invention has been described with reference to specific details of certain embodiments thereof, it is not intended that such details should be regarded as limitations upon the scope of the invention.

Mycobacterial infections, such as those causing tuberculosis, once thought to be declining in occurrence, have rebounded, and again constitute a serious health threat. Tuberculosis (TB) is the cause of the largest number of human deaths attributed to a single etiologic agent with two to three million people infected with tuberculosis dying each year. Areas where humans are crowded together, or living in substandart housing, are increasingly found to have persons affected with mycobacteria. Individuals who are immunocompromised are at great risk of being infected with mycobacteria and dying from such infection. In addition, the emergence of drug-resistant strains of mycobacteria has led to treatment problems of such infected persons.

Many people who are infected with mycobacteria are poor, or live in areas with inadequate healthcare facilities. As a result of various obstacles (economical, education levels, etc.), many of these individuals are unable to comply with the prescribed therapeutic regimens. Ultimately, persistent non-compliance by these and other individuals results in the prevalence of disease. This noncompliance is frequently compounded by the emergence of drug-resistant strains of mycobacteria. Effective compositions and vaccines that target various strains of mycobacteria are necessary to bring the increasing number of tuberculosis cases under control.

Chemotherapy is a standard treatment for tuberculosis. Some current chemotherapy treatments require the use of three or four drugs, in combination, administered daily for two months, or administered biweekly for four to twelve months. Table 1 lists several treatment schedules for standard tuberculosis drug regimens.

**Table 1**

| Treatment Schedules for Standard TB Drug Regimens. | | | | | |
|---|---|---|---|---|---|
| STANDARD DRUG REGIMEN | INDUCTION PHASE Dosing Schedule | DURATION | DRUG | CONTINUATION PHASE Dosing Schedule | DURATION |
| Isoniazid | Daily, DOT | 8 weeks | Isoniazid | 2/week, DOT | 16 weeks |
| Rifampicin | Daily, DOT | 8 weeks | Rifampien | 2/week, DOT | 16 weeks |
| Pyrazinamide | Daily, DOT | 8 weeks | | | |
| Ethambutol or Streptomycin | Daily, DOT | 8 weeks | | | |

Decades of misuse of existing antibiotics and poor compliance with prolong and complex therapeutic regimens has led to mutations of the mycobacterium tuberculosis and has created an epidemic of drug resistance that threatens tuberculosis control world wide. The vast majority of currently prescribed drugs, including the front line drugs, such as isoniazid, rifampin, pyrazinamide, ethambutol and streptomycin were developed from the 1950s to the 1970s. Thus, this earlier development of tuberculosis chemotherapy did not have at its disposal the implications of the genome sequence of *Mycobacterium tuberculosis,* the revolution in pharmaceutical drug discovery of the last decades, and the use of national drug testing and combinational chemistry.

Consequently, the treatments of drug-rosistant *M. tuberculosis* strains, and latent tuberculosis infections, require new anti-tuberculosis drugs that provide highly effective treatments, and shortened and simplified tuberculosis chemotherapies. Moreover, it is desirable that these drugs be prepared by a low-cost synthesis, since the demographics of the disease dictate that cost is a significant factor.

The present invention provides compounds and compositions effective in treatment and prevention of disease caused by microorganisms including, but not limited to, bacteria. In particular, the compounds and compositions of the present invention are effective in inhibiting the growth of the microorganism, *M. tuberculosis.* The coumpounds and compositions of the present invention are intended for the treatment of mycobacteria infections in human, as well as other animals. For example, the present invention may be particularly useful for the treatment of cows infected by *M*. *bovis.*

As used herein, the term "tuberculosis" comprises disease states usually associated with infections caused by mycobacteria species comprising *M*. *tuberculosis* complex. The term "tuberculosis" is also associated with mycobacterial infections caused by mycobacteria other than *M. tuberculosis* (MOTT). Other mycobacterial species include M. *avium-intracellulare, M. kansati, M. fortuitum, M. chelonae, M. leprae. M. africanum,* and *M*. *microti, M. avium paratuberculosis, M. intracellulare, M. scrofulaceum, M. xenopi, M. marinum, M. ulcerans.*

### Second Generation Antibiotics from Ethambutol

A comparative novel library of diamine compounds of Etharnbutol family comprising a modified ethylene linker was prepared starting from commercially available amino alcohol pre-loaded resins.

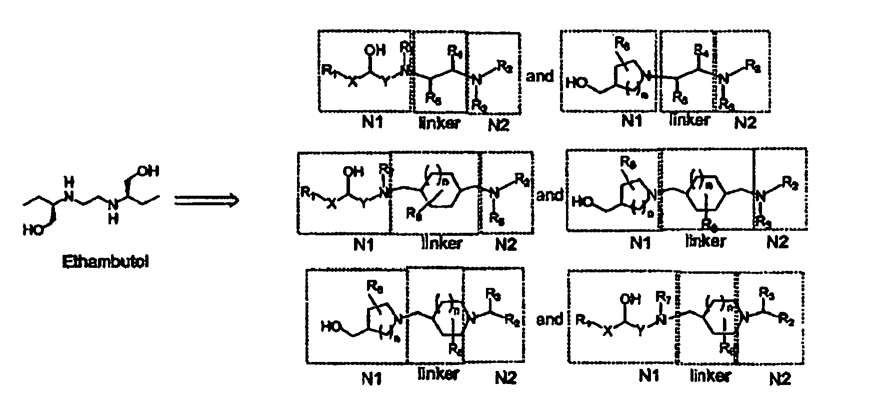

In an effort to enhance the structural diversity of our library of Ethambutol analogs and to assess the influence of a modified linker on the activity of structurally diverse diamines against *M. tuberculosis,* the synthetic scheme to incorporate amino acids into the bridging linker between the two amine components was modified. Use of amino acids was of a special interest since it allowed introduction of another diversity element into the linker (as R4), as well as chirality.

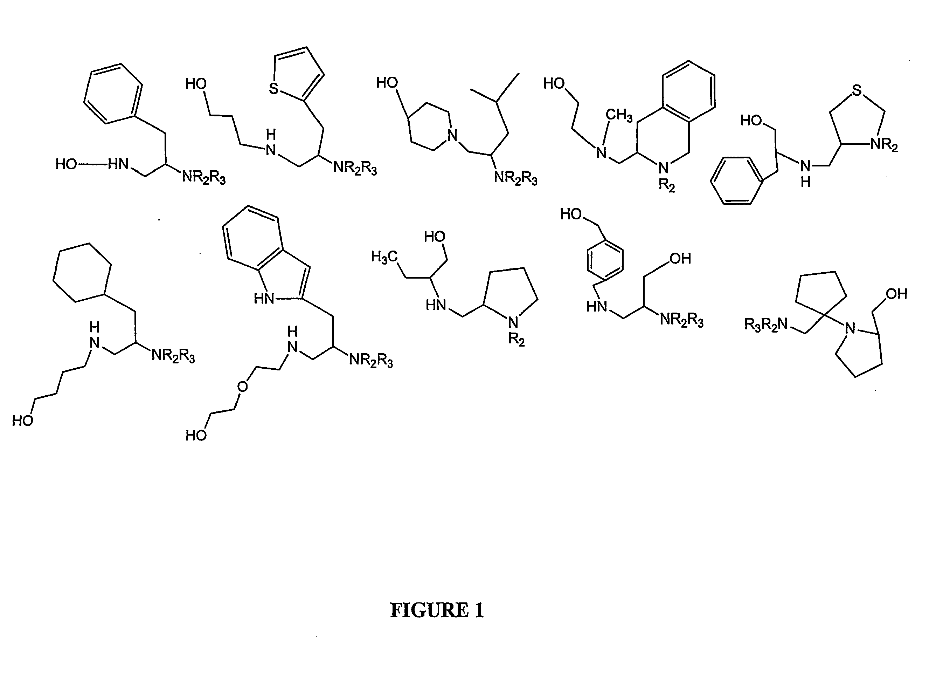

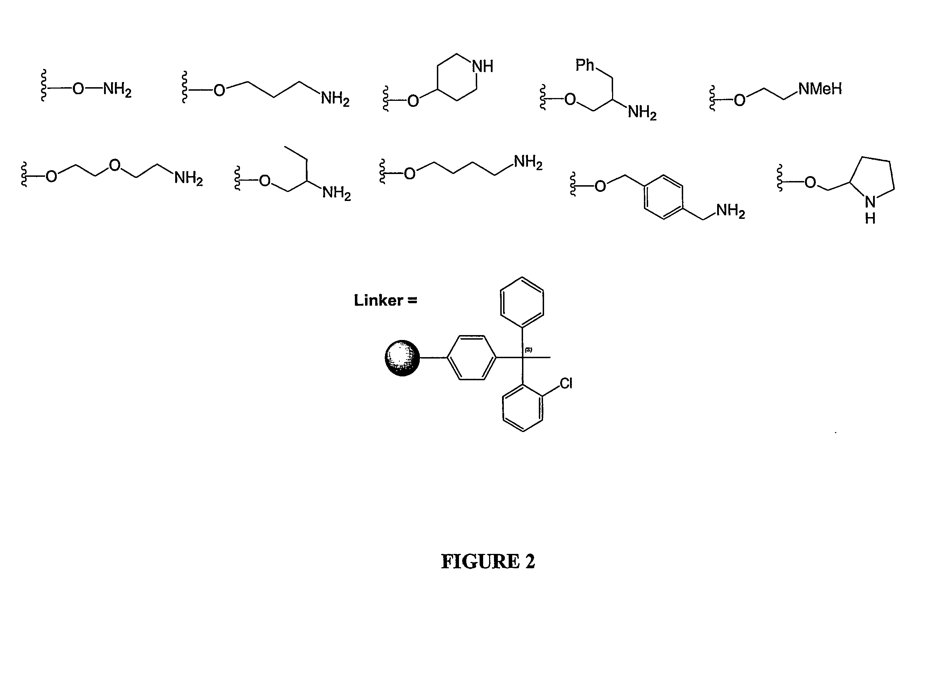

It was crucial for the project to synthesize a sub-library of diamines closely related to Ethambutol by having amino alcohol moiety in the molecule, and yet quite different due to presence an elaborate linker between the two amine atoms (see Figure 1 for representative examples). Also, the fact that the 2-Chlorotrityl resins with pre-loaded amino alcohols are commercially available (Figure 2) and suit well proposed chemistry was very attractive.

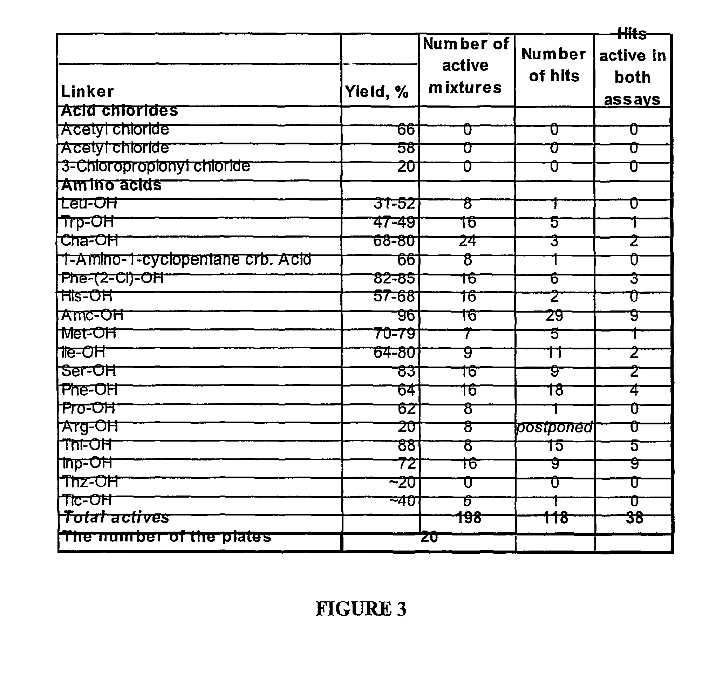

The compounds in the library were prepared on mmol scale in 96-well format in pools of 10 compounds per well (for the vast majority of the plates). Table 1 (Figure 3) summarizes data for the synthesized plates.

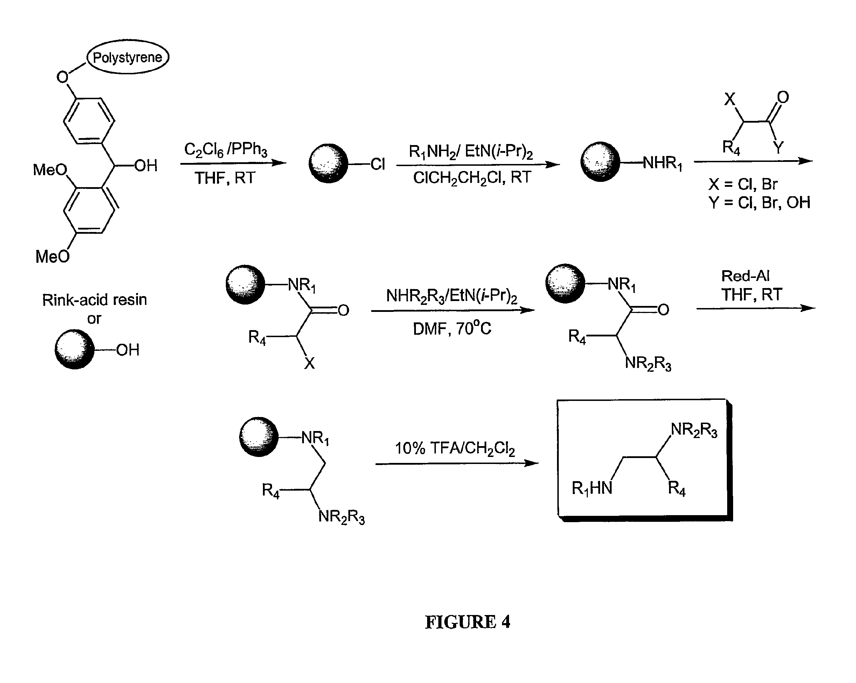

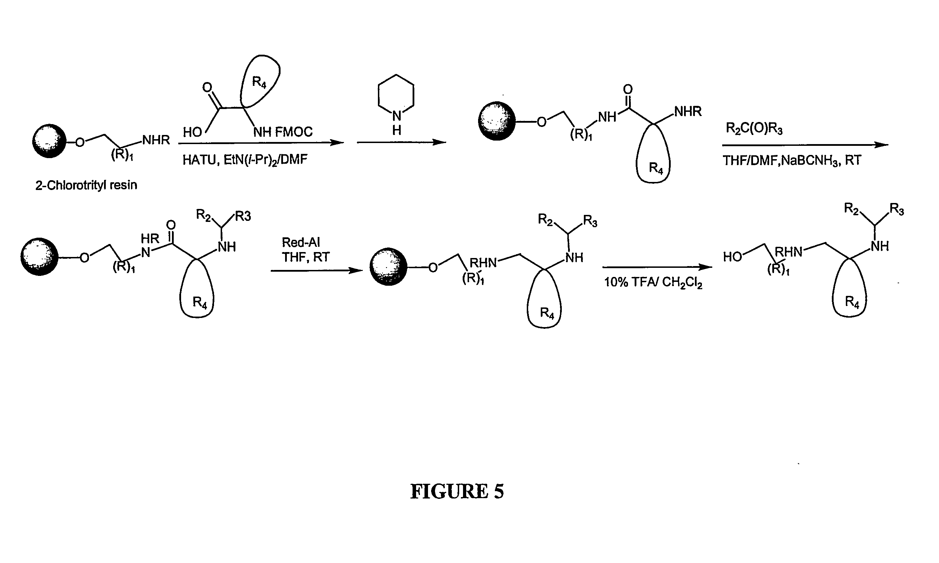

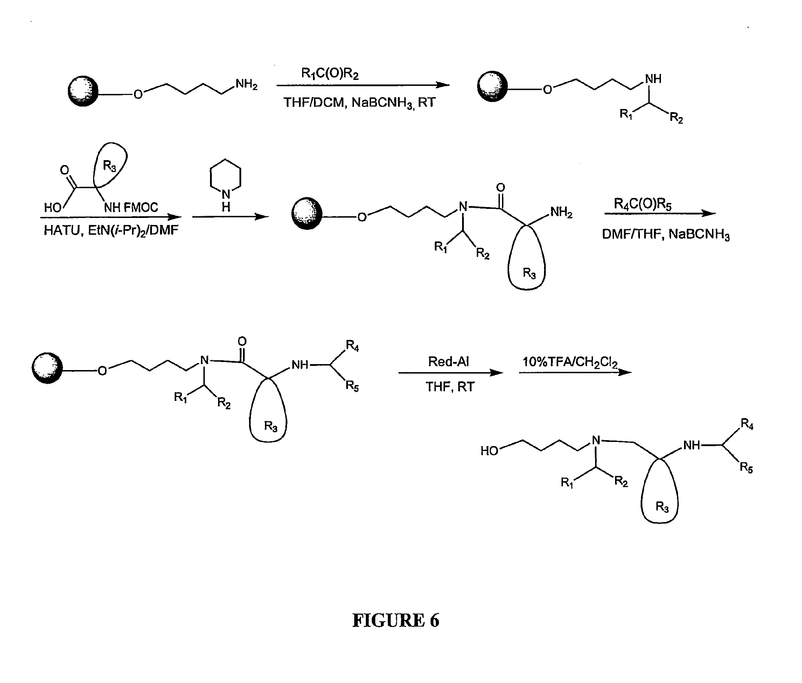

*Solid phase syntheses using amino alcohol pre-loaded resins.* Twenty 96-well plates have been prepared. Four- and five-steps synthetic routes starting from commercially available amino alcohol pre-loaded resins similar to what had been used to create our first 100,000 compound library (Scheme 1, Figure 4), were applied to make targeted diamines (Schemes 2 and 3, Figures 5 and 6 respectively). There are some differences in the syntheses: (1) two first steps of the Scheme 1 are abandoned in Schemes 2 and 3; (2) in the Scheme 1, the second amine is introduced into the molecule as a whole synthon via nucleophilic displacement of Cl-function of the linker, while in the Schemes 2 and 3, it proceeds through modification of the existing amino moiety by carbonyl compounds.

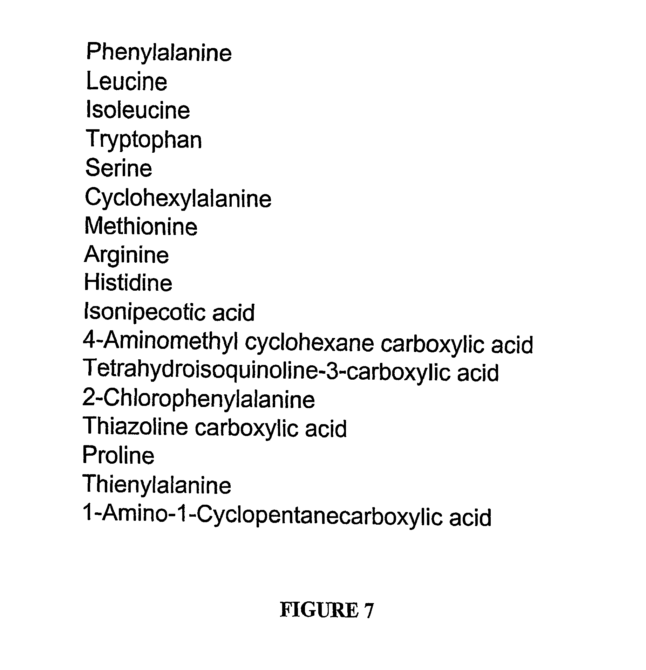

*Scheme 2*. Acylation of the purchased amino alcohol pre loaded 2-Chlorotrityl resins was accomplished *via* peptide coupling with FMOC protected amino acids in presence of HATU (O-(7-Azabenzotriazol-1-yl)-N,N,N,N-tetramethyluronium hexafluorophosphate) and EtN(*iso*-Pr)₂ in DCM/DMF mixture at RT. The reaction was done twice to improve product yields. The list of the amino acids used to create this library is shown in the Table 2 (Figure 7).

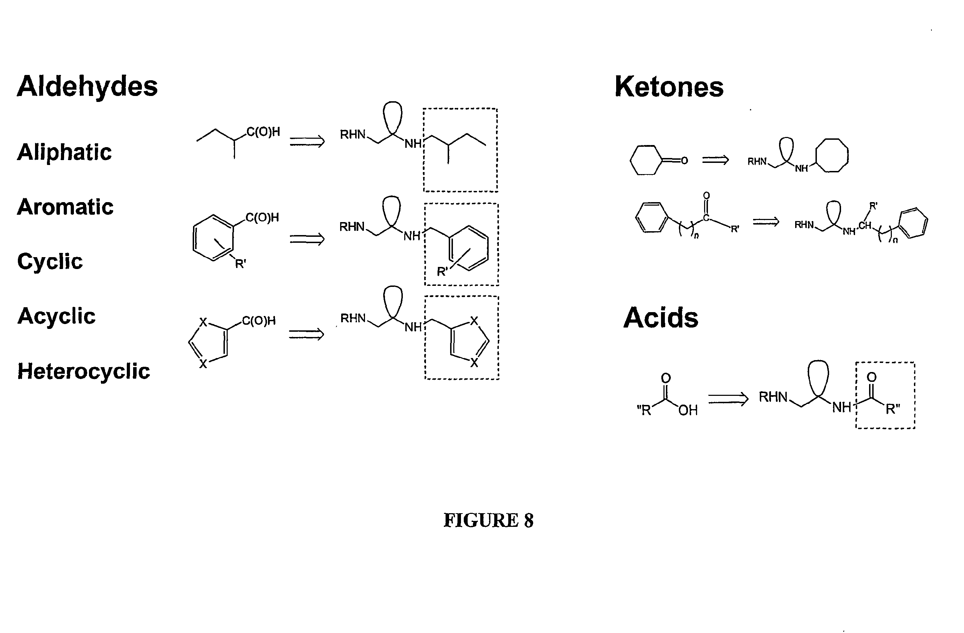

Deprotection (removal of the FMOC group) was carried out by reaction with piperidine at RT. Derivatization of the amino group was achieved by reductive alkylation with 96 various carbonyl compounds, such as aldehydes, ketones, and carboxylic acids, in the presence of NaBCN_{H}3 at RT for 72-96 h. The selection of the carbonyl compounds was made so, that the final diamine products would carry the same or similar types of substituents that had been observed in the hit compounds generated from the previous library of ethambutol analogs, as well as structural diversity (Figure 8). A complete list of the carbonyl compounds used is shown in Table 3 (Figure 8).

Reduction of the aminoethyleneamides into corresponding diamines was carried out using the soluble reducing reagent 65+w% Red-A1 at room temperature. Cleavage of the products from the resin was achieved with a 10% solution of trifluoroacetic acid in dichloromethane resulting in the formation of TFA salts of the diamines.

For library production the acylation step of the synthetic scheme was carried out using a Quest 210 Synthesizer on scale of 0.1-0.15 g of resin per tube. Following the reaction, formed resins were thoroughly washed, dried, and then groups of ten resins were pooled together. A small amount of each resin (∼0.05g) was archived prior to pooling to facilitate re-synthesis and deconvolution of actives.

Deprotection of the FMOC group, addition of the carbonyl component, reduction, and cleavage were carried out in 96-well reaction blocks using the Combiclamps system by Whatman Polyfiltronics or the FlexChem system by Robbins Scientific. A suspension of the pooled resins in 2:1 mixture of DCM/THF was evenly distributed into one reaction plate resulting in approximately 10 mg of the resin per well. The 96 diverse carbonyl compounds were arrayed in one 96-well plate template and added, one carbonyl compound per well, to each individual pool of ten resins, resulting in an anticipated 960 diamines produced per plate. Reduction was carried out in the same format and cleavage and filtering into storage plates was followed by evaporation of the TFA prior to biological assay.

Quality assessment of the prepared library of diamines was done by Electrospray Ionization mass spectrometry using two randomly selected rows (16 samples) per plate, 17% of the total number. Successful production of a compound was based on an appearance of a molecular ion of the calculated mass. Depending on the amino acid that had been used for the synthesis, the percentage of the predicted ions were observed and therefore the predicted compounds were formed, varied from 31-96% (Table 1, Figure 3). Based on MS analysis, out of targeted 15,360 compounds, 7,500 diamines were actually formed. Amino acids such as aminomethylcylcohexyl carboxylic acid, thienylalanine, or phenylalanine produced desirable compounds with good yields (88-96%). At the same time, some amino acids, such as arginine, tetrahydroisoquinoline carboxylic acid, and thiazolidine carboxylic acid did not lead to the corresponding products.

*Schemes 3*. Success in preparation of ethambutol analogs with modified linker encouraged us to attempt synthesis of another sub-library of diamines using commercially available amino-alcohol pre-loaded resins (Scheme 3, Figure 6). This route yields diamine compounds that are similar to those produced by the (Scheme 2, Figure 5) but that also possess desirable substituents at the first nitrogen atom.

We have illustrated this method by starting from commercially available 1,4-aminobutanol pre-loaded resin. We prepared five plates using five amino acids Phe, Amc, Cha, Trp, and Inp (Table 1) that gave the best preliminary results in the screening assays (see number of hits in the Table 1). The very first step was derivatization of the amino group *via* reductive alkylation by 10 carbonyl compounds (cyclooctanone, 4-benzyloxybenzaldehyde, (S)-citronellal, myrtenal, tetrahydro-4H-pyran-4-one, norcamphor, 4-(4-hydroxyphenyl)2-butanone, geranylacetone, 2-decalone, 2-adamantanone) in presence of NaBCNH₃ at RT. The following steps were carried out in a similar fashion as we reported earlier for the Scheme 2.

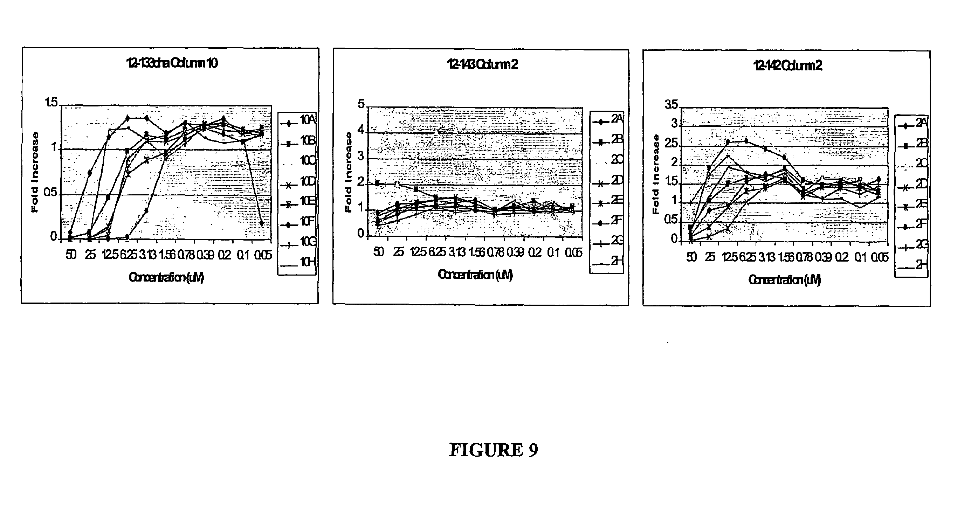

*Screening the library against Tuberculosis and deconvolution of the active mixtures.* A high-throughput assay with recombinant mycobacteria containing a promoter fusion of luciferase to Rv0341, as well as the MICs, has been used to screen this new compound library of ethambutol analogs, Figure 9.

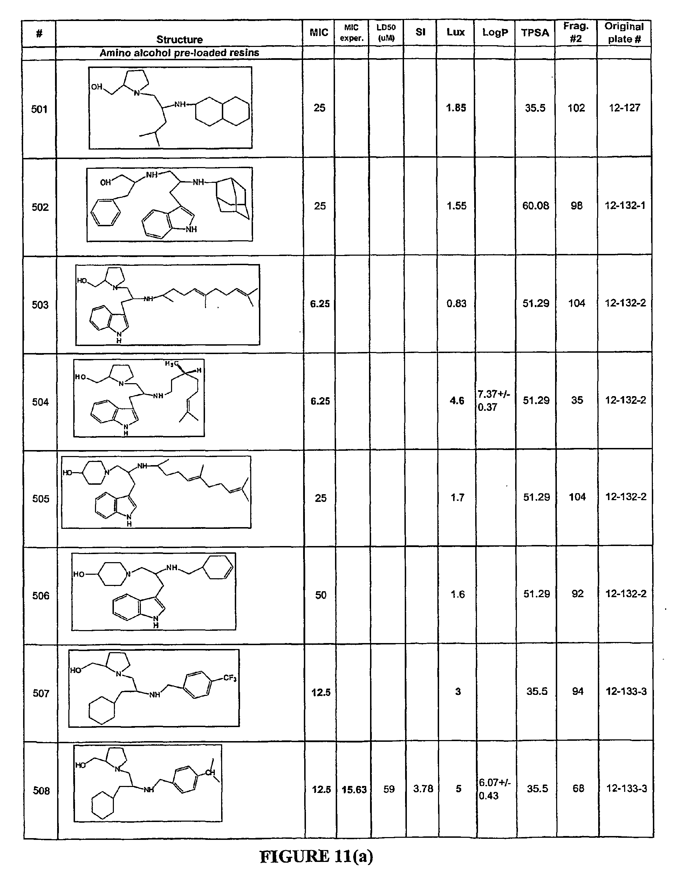

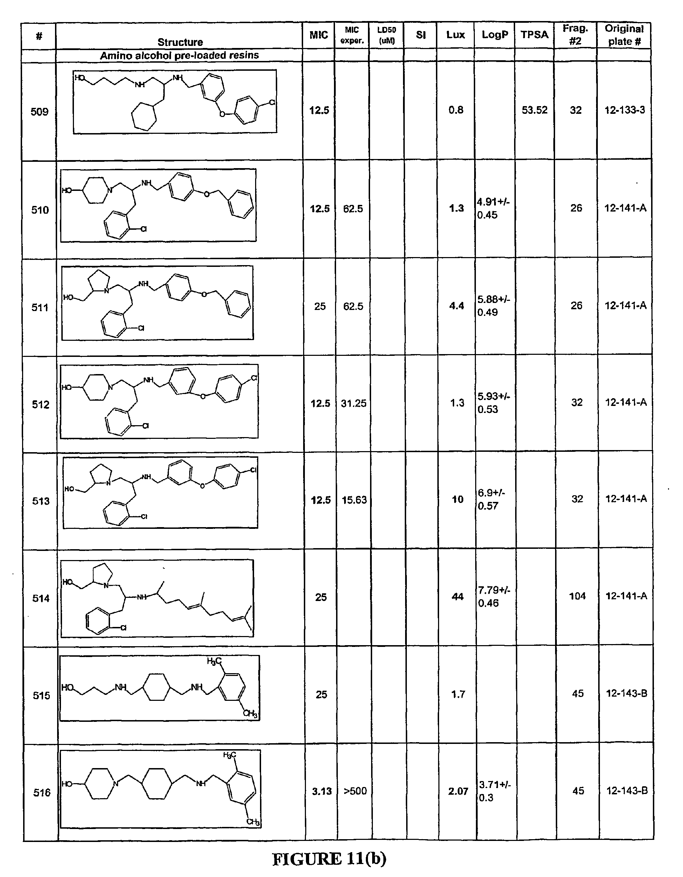

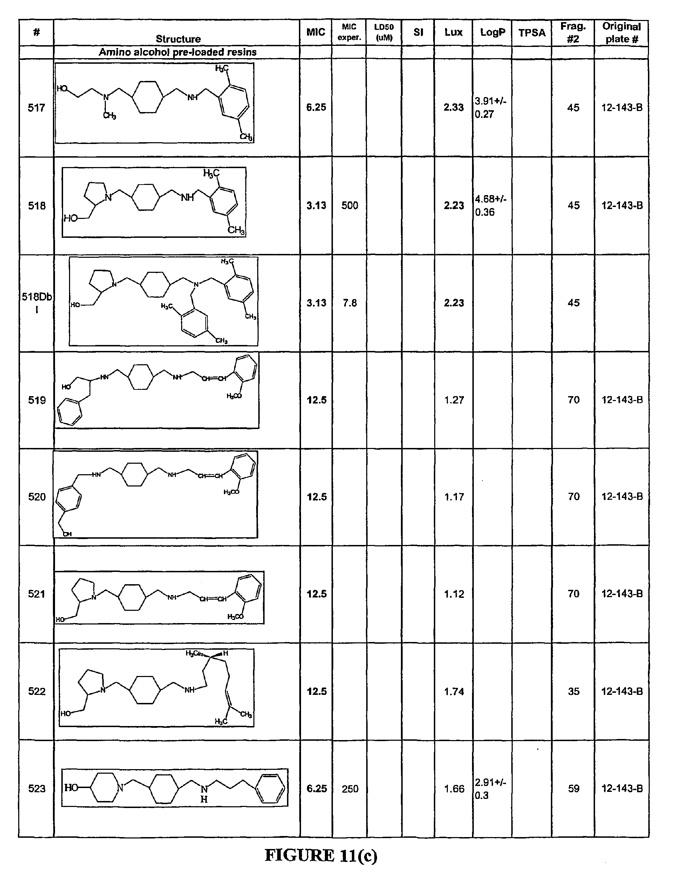

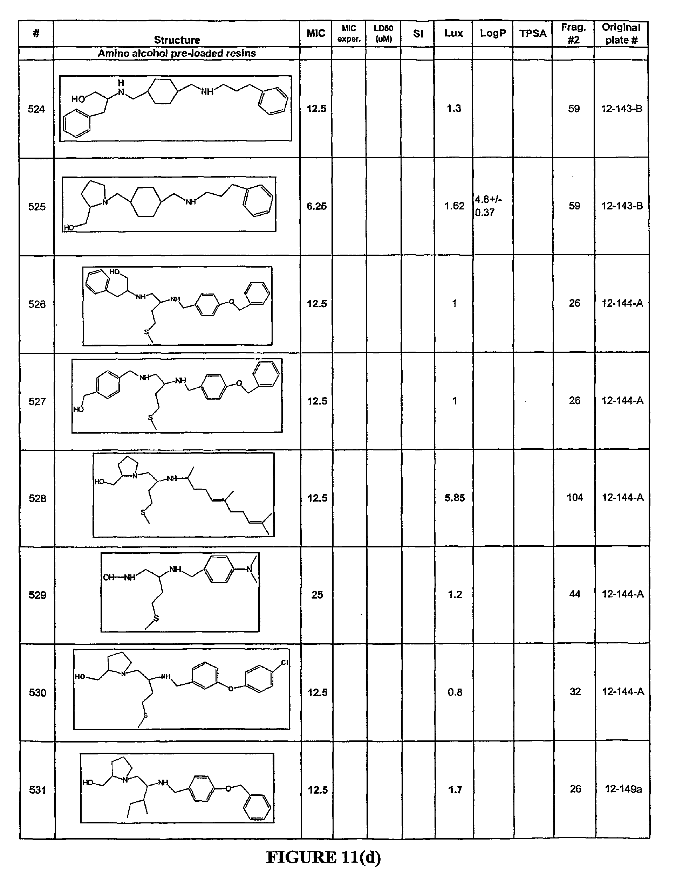

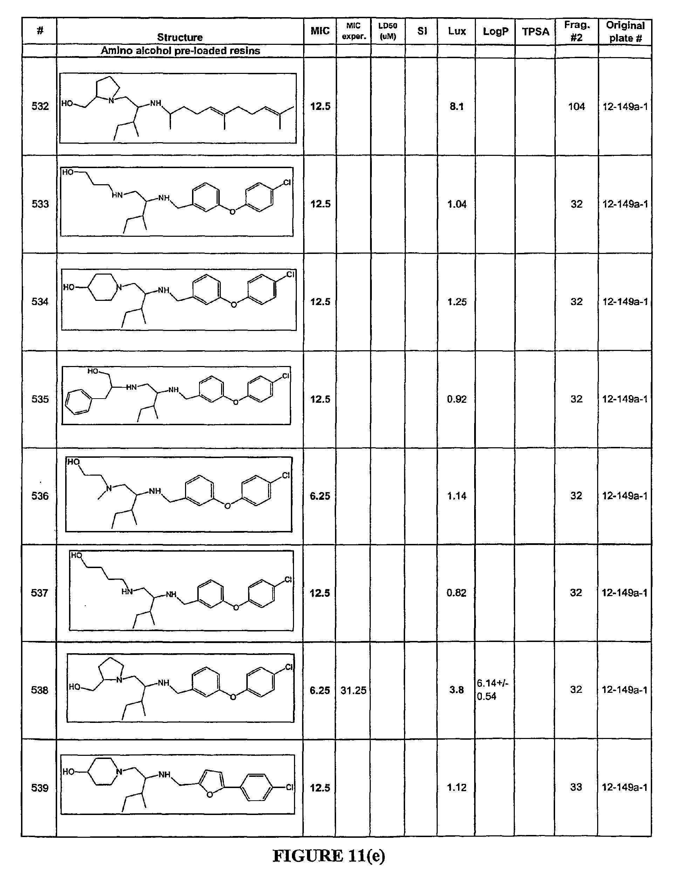

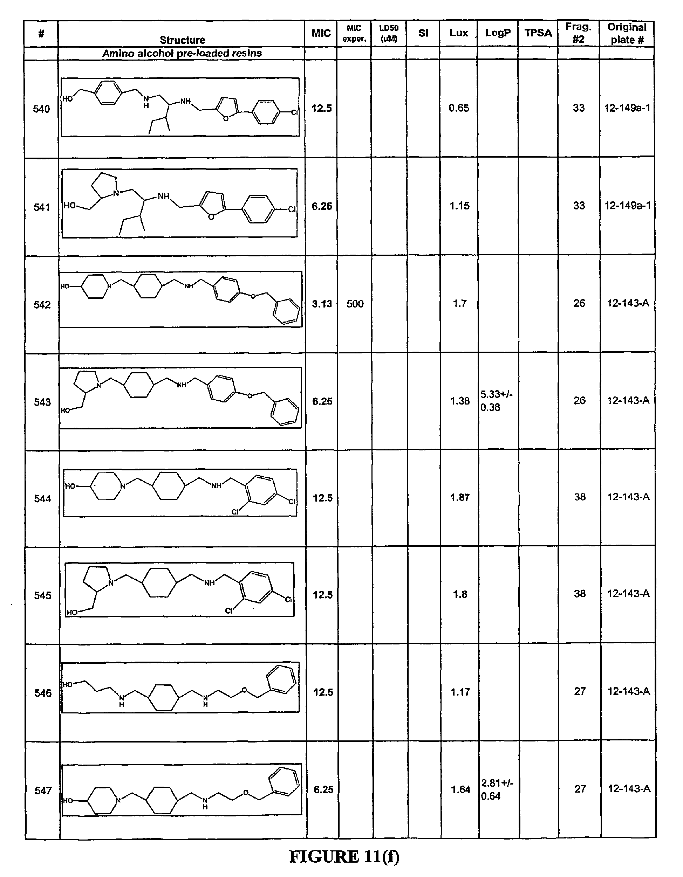

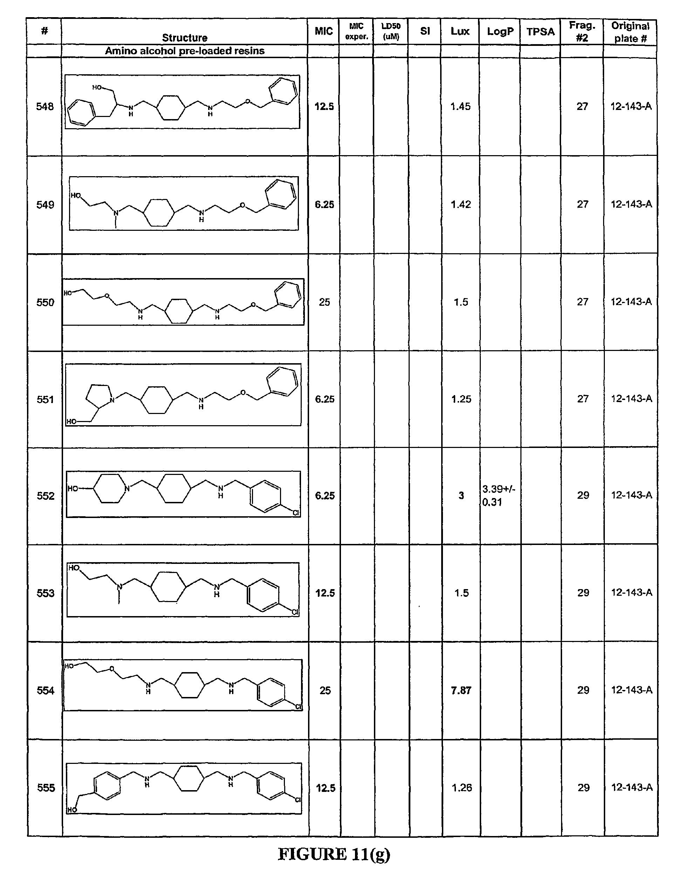

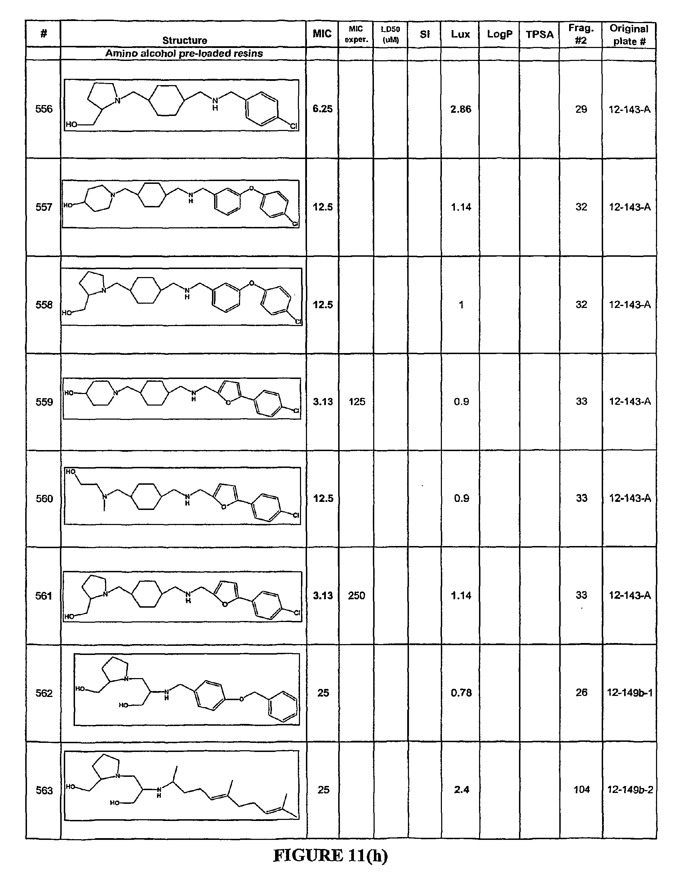

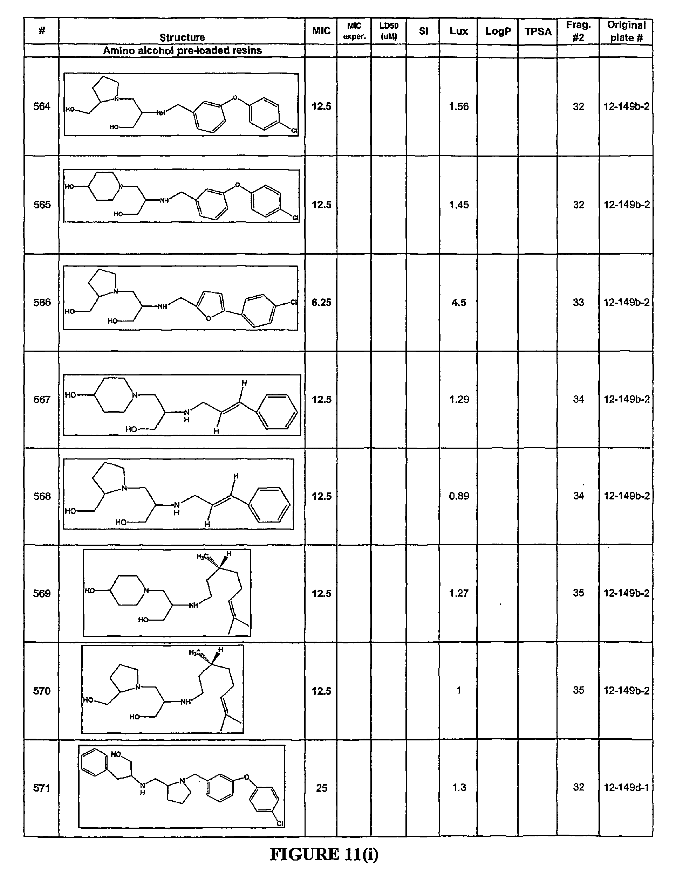

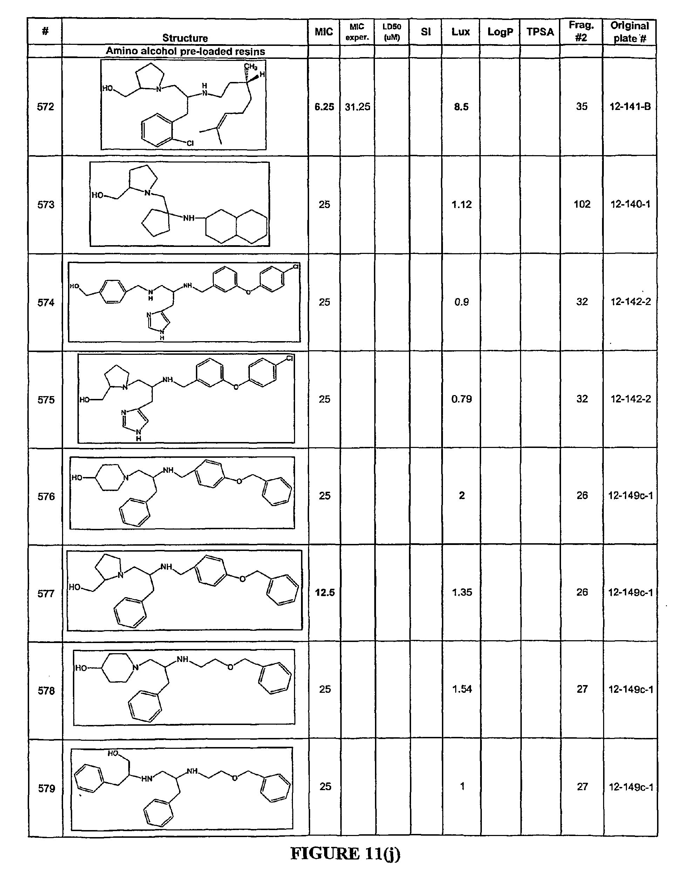

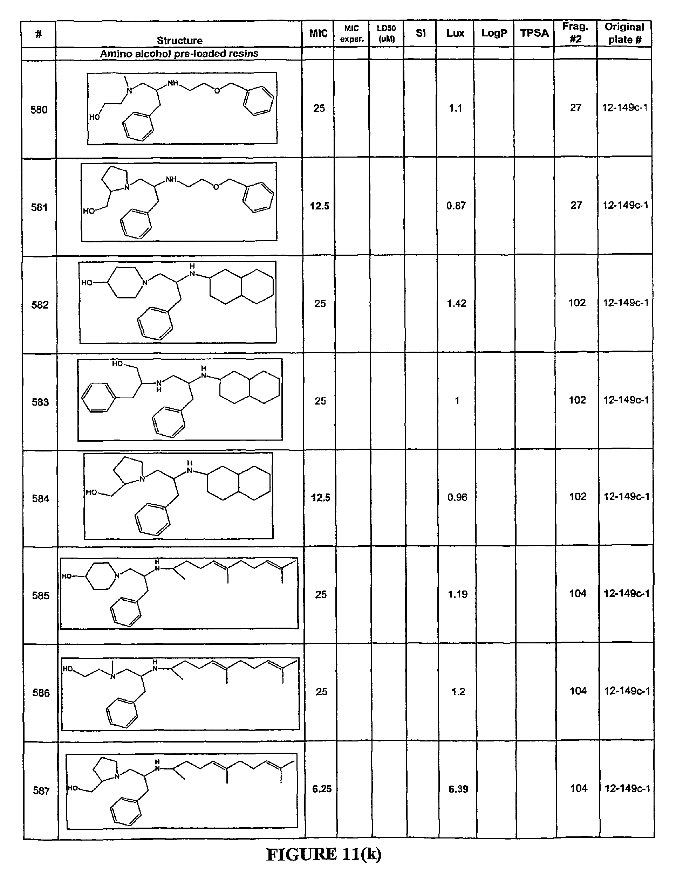

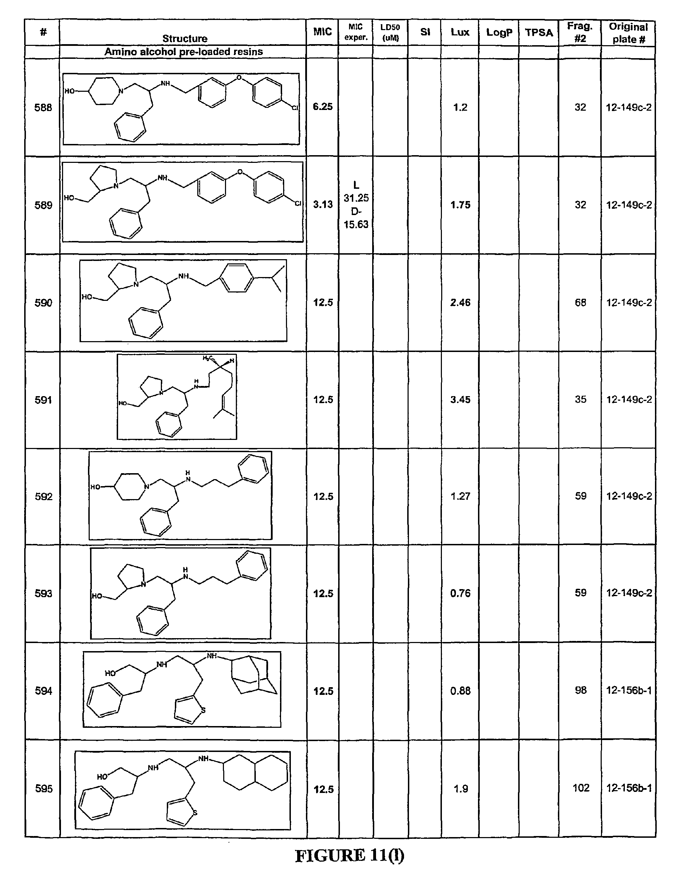

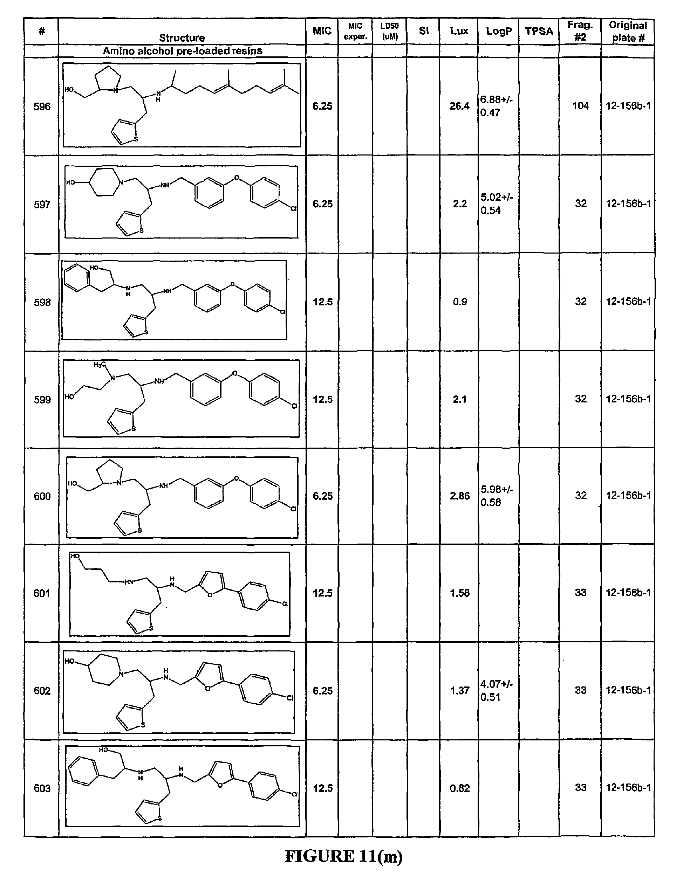

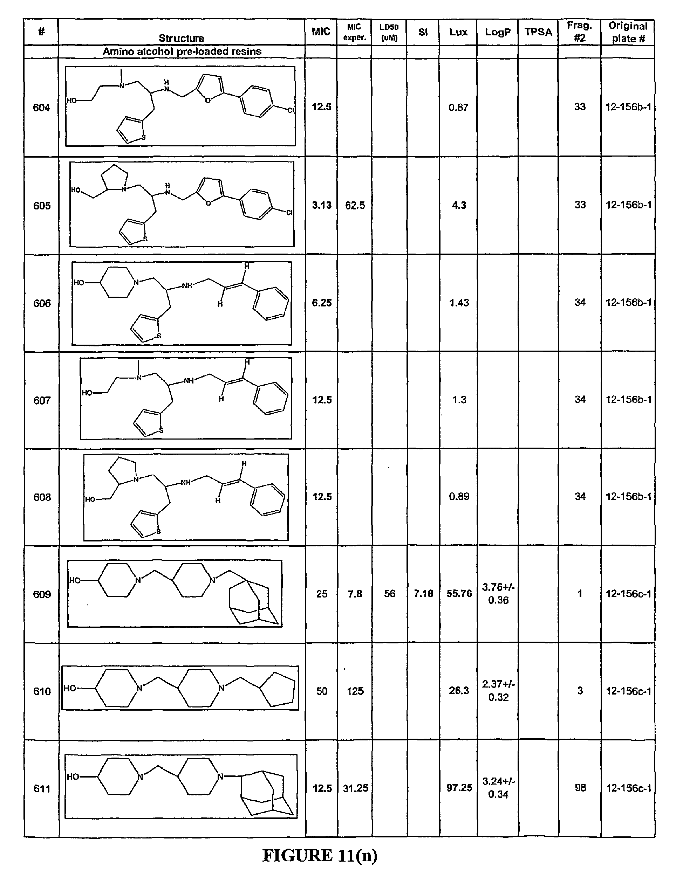

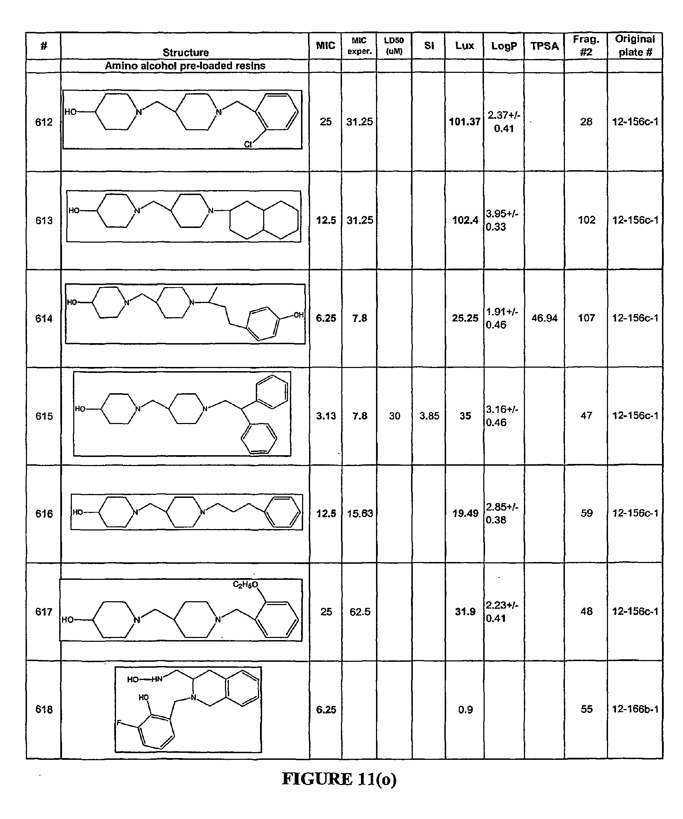

198 compound mixtures have shown to exhibit anti-TB activity, Table 1 (activity at <12.5 uM in the HTS Luc assay and/or with an MIC of <12.5 uM), have been selected for deconvolutions. Deconvolutions of all 198 compound mixtures were performed by the discrete re-synthesis of the diamine compounds in 96-well format using stored archive resins (prior pooling them together) and the same synthetic Schemes 2 and 3. The same screening tests were used for every deconvoluted plate. Few carbonyl compounds have been identified as potent synthons contributing to the anti-TB activity (Figure 5). Performed deconvolutions revealed 118 hits of novel structures as potent anti-TB compounds (Table 4), 38 of those compounds were proven to be active in both assays. Figure 11 provides a list of hit compounds and their structures.

### Formulations

Therapeutics, including compositions containing the substituted ethylene diamine compounds of the present invention, can be prepared in physiologically acceptable formulations, such as in pharmaceutically acceptable carriers, using known techniques. For example, a substituted ethylene diamine compound is combined with a pharmaceutically acceptable excipient to form a therapeutic composition.

The compositions of the present invention may be administered in the form of a solid, liquid or aerosol. Examples of solid compositions include pills, creams, soaps and implantable dosage units. Pills may be administered orally. Therapeutic creams and anti-mycobacteria soaps may be administered topically. Implantable dosage units may be administered locally, for example, in the lungs, or may be implanted for systematic release of the therapeutic composition, for example, subcutaneously. Examples of liquid compositions include formulations adapted for injection intramuscularly, subcutaneously, intravenously, intraarterially, and formulations for topical and intraocular administration. Examples of aerosol formulations include inhaler formulations for administration to the lungs.

A sustained release matrix, as used herein, is a matrix made of materials, usually polymers, which are degradable by enzymatic or acid/base hydrolysis, or by dissolution. Once inserted into the body, the matrix is acted upon by enzymes and body fluids. The sustained release matrix is chosen desirably from biocompatible materials, including, but not limited to, liposomes, polylactides, polyglycolide (polymer of glycolic acid), polylactide co-glycolide (coplymers of lactic acid and glycolic acid), polyanhydrides, poly(ortho)esters, polypeptides, hyaluronic acid, collagen, chondroitin sulfate, carboxylic acids, fatty acids, phospholipds, polysaccharides, nucleic acids, polyamino acids, amino acids such as phenylalanine, tyrosine, isoleucine, polynucleotides, polyvinyl propylene, polyvinylpyrrolidone and silicone. A preferred biodegradable matrix is a matrix of one of either polylactide, polyglycolide, or polylactide co-glycolide.

The dosage of the composition will depend on the condition being treated, the particular composition used, and other clinical factors, such as weight and condition of the patient, and the route of administration. A suitable dosage may range from 100 to 0.1 mg/kg. A more preferred dosage may range from 50 to 0.2 mg/kg. A more preferred dosage may range from 25 to 0.5 mg/kg. Tablets or other forms of media may contain from 1 to 1000 mg of the substituted ethylene diamine. Dosage ranges and schedules of administration similar to ethambutol or other anti-tuberculosis drugs may be used.

The composition may be administered in combination with other compositions and procedures for the treatment of other disorders occurring in combination with mycobacterial disease. For example, tuberculosis frequently occurs as a secondary complication associated with acquired immunodeficiency syndrome (AIDS). Patients undergoing AIDS treatment, which includes procedures such as surgery, radiation or chemotherapy, may benefit from the therapeutic methods and compositions described herein.

The following specific examples will illustrate the invention as it applies to the particular synthesis of the substituted ethylene diamine compounds, and the *in vitro* and *in vivo* suppression of the growth of colonies of *M*. *tuberculosis.* In additiona, the teachings of R. Lee et al. J. Comb. Chem 2003, 5, 172-187 are hereby incorporated by reference in their entirety. It will be appreciated that other examples, including minor variations in chemical procedures, will be apparent to those skilled in the art, and that the invention is not limited to these specific illustrated examples.

### EXAMPLE I

### Generating the Diamine Library from commercially available amino alcohol pre-loaded resins.

*General Methods:* All reagents were purchased from Sigma-Aldrich. Amino alcohol pre-loaded resins were purchased from NovaBiochem. Solvents acetonitrile, dichloromethane, dimethylformamide, ethylene dichloride, methanol, and tetrahydrofuran were purchased from Aldrich and used as received. Solid phase syntheses were performed on Quest 210 Synthesizer (Argonaut Technologies) and combinatorial chemistry equipment (Whatman Polyfiltronics and Robbins Scientific). Evaporation of the solvents was done using SpeedVac AES (Savant). Mass spectra data were obtained by Electrospray Ionization technique on Perkin Elmer/Sciex, API-300, TQMS with an autosampler.

### Scheme 2. Description of the Process.

The acylation step was carried out in 5 ml tubes using the Quest 210 Synthesizer. Removal of the FMOC group, reductive alkylation reaction with carbonyl compounds, the reduction with Red-Al, and the cleavage from the solid support were carried out in 96-deep (2ml) well, chemically resistant plates.

### Step 1. Acylation of amino alcohol pre-loaded resins with amino acids.

Each tube was loaded 0.150 g of corresponding resin (coverage of 0.3-1.0 mmol/g), and all resins were pre-swollen in DCM for 1.5 h and filtered*. If Fmoc protected resin was used:* the resin was stirred with 2.5 ml of 20% solution of piperidine in DMF for 10 min, filtered, and washed with 2.5 ml of DMF. The procedure was repeated, but the stirring time was 20 min. After that all resins were filtered, washed with DMF (1x2.5 ml) and DCM (2x3ml). Each tube was charged with 1 ml of dichloromethane. Amino acids, 0.38mmol in 1 ml of DMF (2,5 mol excess to loaded resin) were mixed with HATU, 0.3 mmol, 011 g in 0.5 ml of DMF (2 mol excess to loaded resin) and allowed to stay for 15-20 min. Then 1.5 ml of mixture acid-HATU were added to each tube following by the addition of solution of 1.5 mmol, 0.26 ml (10 mol excess to loaded resin) of EtNPr₂ in 0.5 ml of dichloromethane. Reaction carried out 8 h at 45°C and 6-8 h at room temperature. After the reaction was complete, the resins were filtered, washed with 1:1 1 mixture of DMF and dichloromethane (1x3ml), dichloromethane (1x3ml), and acylation was repeated with the same amount of reagents. At the end, the resins were filtered, washed with 1:1 mixture of DMF and dichloromethane (1x3ml), methanol (3x3ml), sucked dry (on Quest) for 30 min and transferred into vials (one resin per vial), and dried in a desiccator under vacuum for 1h. After this step all resins were subjected for quality control using MS spectra.

### Step 2. Alkylation of the amino group.

*Deprotection.* Ten prepared resins from the first three steps were pooled together, leaving approximately 0.03 g of each in the individual vials for all necessary deconvolutions. A suspension of the resin mixture (0.08 - 1.0 g) in 100 ml of a 2:1 mixture of dichloromethane and THF was distributed into two 96-well filterplates and filtered using a filtration manifold. The reaction plates were transferred into combiclamps, and 0.2 ml of 20% solution of piperidine in DMF was added to remove Fmoc protecting group and allowed to stay for 10 min. After 10 min plate was filtered, washed with 0.2 ml of DMF, and deprotection was repeated with 0.2 ml of 20% solution of piperidine in DMF and allowed to stay for 20 min. After that plate was filtered, washed with DMF (0.2 ml per well) and dichloromethane (2x0.5 ml per well).

*Reaction with the carbonyl compounds.* Each well in row A on the reaction plate was charged with 0.1 ml of dichloromethane, 0.08ml of ∼ 1.0M solution of appropriate acid in DMF from master plate, 0.05 ml DMF solution of PyBrop, (0.012 g, 0.025 mmol, 2.5 mol excess to loaded resin) and 0.05 ml of EtN*i*Pr₂ in dichloromethane (0.017 ml, 0.10 mmol, 10 mol excess to loaded resin). Each well in rows B through H was charged with 0.1 ml of THF, 0.160 ml of ∼1.0 M solution of appropriate aldehyde or ketone in DMF from master plate and allowed to react for 30 min. After 30 min 0.075 ml (0.075 mmol) of 1.0 M solution of NaBCNH₃ in THF were added. The reaction plates were sealed and kept at RT for 72h. At the end, the resins were filtered, washed with THF, DCM (1x1ml), methanol (2x1ml) and dried in desiccator under vacuum for 3h.

### Step 3. Reduction with Red Al.

The reaction plates were placed into combiclamps. A 1:6 mixture of Red-Al (65+ w% in toluene) and THF was added, 0.6 ml per well (0.28mmol of Red-Al per well), and allowed to react for 4 h. After the reaction completion the resins were filtered, washed with THF (2x1m1), methanol (3x1ml) and dried in the filtration manifold.

### Step 4. Cleavage.

This step was carried out using a cleavage manifold. The reaction plates (placed on the top of the collection plates in this manifold) were charged with a 10:88:2 mixture of TFA, dichloromethane, and triisopropylsilane, 0.5 ml per well. After 15 min, the solutions were filtered and collected into proper wells of the collection plates. The procedure was repeated. Solvents were evaporated on a speedvac, and the residual samples were ready for testing.

### Scheme 3. Description of the Process.

The reductive alkylation step of 4-aminobutan-1 ol resin and the acylation step were carried out in 5 ml tubes using the Quest 210 Synthesizer. Removal of the FMOC group, reductive alkylation reaction with carbonyl compounds, the reduction with Red-Al, and the cleavage from the solid support were carried out in 96-deep (2ml) well, chemically resistant plates.

### Step 1. Reductive alkylation of 4-Aminobutan-l-ol resin.

A suspension of the resin (coverage of 0.3-1.0 mmol/g), 1.0 g (up to 1.0 mmol), in 30 ml of 2:1 mixture of dichloromethane and THF was disitrubuted into 10 tubes, 3 ml per tube and filtered, than each tube was charged with 0.10 g of resin. Resin was pre swollen in DCM for 1.5 h and filtered. Each tube were loaded with 1.5 ml 1,2-dichloroethane, 0.3 mmol (3 mol excess) of corresponded aldehyde or ketone (alkylating reagent) and allowed to react for 30 min. After that 0.3 mmol (0.3 ml) of 1 M solution of NaBCNH₃ in THF were added and reaction was carried out at RT for 48 h. When reaction was completed, all tubes were filtered, washed with THF (2x3ml), MeOH (3x3ml) and sucked dry (on Quest for ∼30min).

### Step 2. Acylation with amino acids.

All tubes were pre washed with DCM twice. Each tube was charged with 1 ml of dichloromethane. Amino acids, 0.25 mmol in 1 ml of DMF (2,5 mol excess to loaded resin) were mixed with HATU, 0.2 mmol, 0.076 g in 0.5 ml of DMF (2 mol excess to loaded resin) and allowed to stay for 15-20 min. Then 1.5 ml of mixture acid-HATU were added to each tube following by the addition of solution of 1.0 mmol, 0.17 ml (10 mol excess to loaded resin) of EtN*i*Pr₂ in 0.5 ml of dichloromethane. Reaction carried out 8 h at 45°C and 6-8 h at room temperature. After 16 h the resins were filtered, washed with 1:1 mixture of DMF and dichloromethane (1x3ml), dichloromethane (1x3ml) and acylation was repeated with the same amount of reagents. At the end, the resins were filtered, washed with 1:1 mixture of DMF and dichloromethane (1x3ml), methanol (3x3ml), sucked dry (on Quest) for 30 min and transferred into vials (one resin per vial), and dried in a desiccator under vacuum for 1h. After this step all resins were subjected for quality controlusing MS spectra.

*All following reaction steps, - alkylation of the amino group (Step 3), reduction with Red-Al (Step 4), and Cleavage (Step 5), - were carried as they were described for the Scheme 2 of this Application.*

### EXAMPLE 2

### Deconvolution

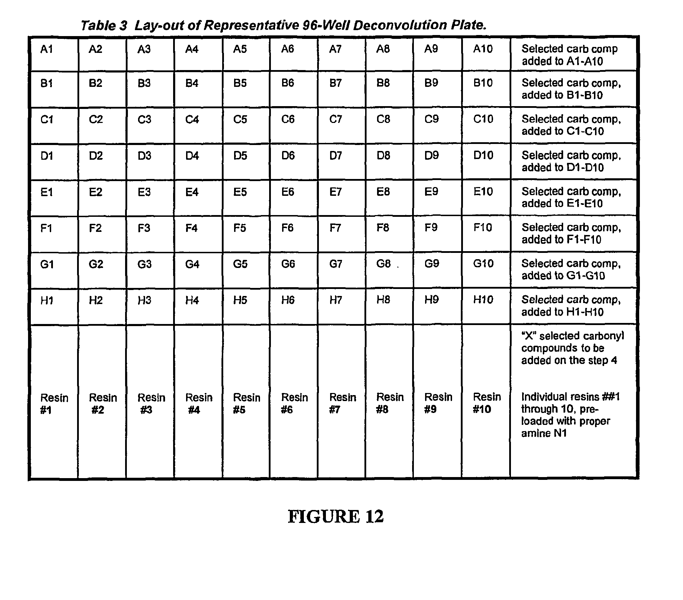

Deconvolution of the active wells was performed by re-synthesis of discrete compounds, from the archived FMOC-protected □ -aminoacetamide resins (10 resins, 0.05-0.10g each), which were set aside at the end of the acylation step before the pooling. Each resin was assigned a discrete column (1, or 2, or 3, etc.) in a 96-well filterplate, and was divided between X rows (A, B, C, etc), where X is the number of hits discovered in the original screening plate. To each well, in a row, a selected carbonyl compound (present in the hit) was added along with other required reagents: the first selected carbonyl compound was added to the resins in the row "A", the second carbonyl compound- to the resins in the row "B", the third carbonyl compound- to the resins in the row "C", etc. A lay-out of a representative 96-well deconvolution plate is shown in Table 3, Figure 12.

The reaction plates were sealed and kept at RT for 72h. At the end, the resins were filtered, washed with THF, DCM (1x1ml), methanol (2x1ml) and dried in desiccator under vacuum for 2h. Reduction and cleavage were performed according to steps 5 and 6 of the synthetic protocol. The product wells from the cleavage were analyzed by ESI-MS (Electrospray Ionization Mass Spectroscopy) to ensure the identity of the actives, and were tested in the MIC assay.

### Solid Phase Synthesis of Selected Substituted Ethylenediamines Using the Quest 210 Synthesizer.

The solid phase protocol described above for generating a library of diamine compounds was applied to the scale-up synthesis of the selected substituted ethylenediamine compounds. Here, all reaction steps, from the acylation of the commercially available amino alcohol pre-loaded resins to the cleavage of the final product, were carried out using Quest instrument only, which allowed for up to twenty parallel reactions. Purification of all crude samples was done by Flash Chromatography on CombiFlash (Isco, Inc.) to yield desirable products in purity greater than 90%. Here, the synthesis of one of the active compounds, 1-(2-{[3-(4-chlorophenoxy)benzyl]amino}-3-phenylpropyl)piperidin-4-ol, is described below as an example.

The preparation of 1-(2-{[3-(4-chlorophenoxy)benzyl]amino}-3-phenylpropyl)piperidin-4-ol, compound 588.

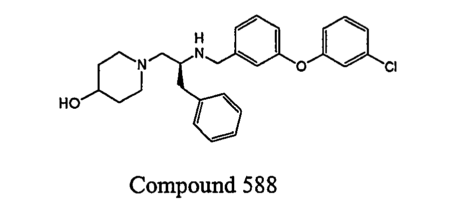

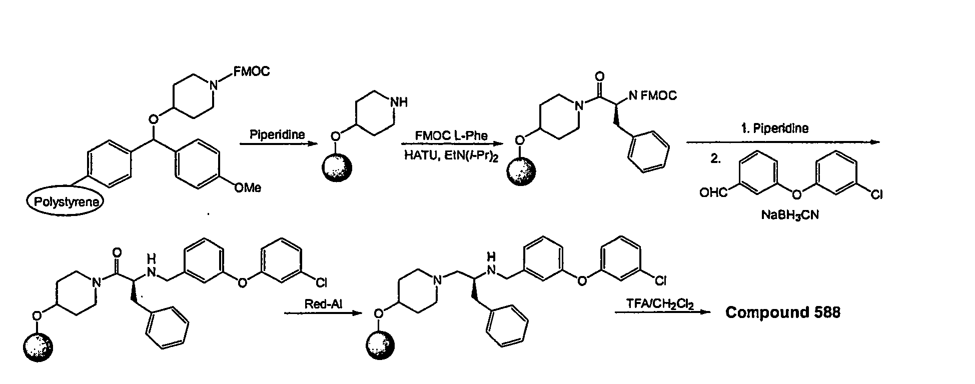

### Representative synthesis of active compounds.

*Removal of FMOC protective group.* Commercially available resin (N-FMOC-piperidinyl-4-oxy)-(4-methoxyphenyl)methyl polystyrene, coverage (linker) 0.88 mmol/g (0.4 g, 0.35 mmol), was placed into one of the 10 ml tubes of Quest 210 Synthesizer. A solution of piperidine (1.5 ml) in DMF (6 ml) was added and stirred for 30 min, filtered, washed with DMF (1x6 ml), and the addition of piperidine was repeated. The resin was washed with DMF (1x8 ml) and DCM (2x8ml).

*Acylation with FMOC protected LPhenylalanine .* The resin was pre-washed with 5 ml of DCM for 20 min. FMOC L-Phenylalanine, (.0341 g, 0.88 mmol) in 1 ml of DMF (2,5 mol excess to loaded resin) were mixed with HATU (0.33 g, 0.88 mmol) in 3 ml of DMF, and added to the tube following by the addition of solution of 0.6 ml of EtN*i*Pr₂. Reaction was carried at RT for 20 h. After the reaction was complete, the resin was filtered, washed with 1:1 mixture of DMF and dichloromethane (1x6ml), dichloromethane (1x6ml), and acylation was repeated with the same amount of reagents. At the end, the resins were filtered, washed with 1:1 mixture of DMF and dichloromethane (2x6ml).

*Removal of FMOC protective group.* A solution of piperidine (1.5 ml) in DMF (6 ml) was added to the resin and formed suspension was stirred for 30 min, filtered, washed with DMF (1x6 ml), and the addition of piperidine was repeated. The resin was washed with DMF (1x8 ml) and methanol (2x8ml), and sucked dry inder Ar for 20 min.

*Reaction with a carbonyl compound.* The resin was pre-washed with THF for 30 min, filtered, and charged with 6 ml of THF. 3-(4-Chlorophenoxy)-benzaldehyde (0.280 ml, 1.00 mmol) was added followed by addition of 1.0 M solution of NaBCNH₃ in THF (1 ml, 1 mmol) after 30 min. The reaction was allowed to proceed at RT for 72h. At the end, the resin was filtered, washed with THF (1x6ml) and MeOH (2x6ml), and dried under Ar for 30 min.

Reduction with Red-Al. The resulted resin in a tube was pre-washed with anhydrous THF (2x6ml) and filtered. The tube was charged with 5 ml of anhydrous THF followed by addition upon stirring commercially available Red-Al as 65+% in toluene (1 ml, 3.2 mmol). After 4 h the resin was filtered, washed with THF (2x1ml) and MeOH (3x1ml) (addition of MeOH should proceed with caution!), and dried under Ar for 10 min.

Cleavage. For this last step of the synthesis the tube with the resin was charged with DCM (8 ml) and TFA (1 ml) and formed bright red suspension was allowed to stir for 30 min. The resin was filtered and *the filtrate was collected* into a collection tube. The procedure was repeated. DCM and excess of TFA were evaporated on a speedvac. Crude 1-(2-{[3-(4-chlorophenoxy)benzyl]amino}-3-phenylpropyl)piperidin-4-ol (in a form of trufluoroacetate salt) was purified by Flash Chromatography on CombiFlash (Isco) using following conditions: pre-loaded silica gel column, 12 g, flow 15 ml/min, 25 min run, gradient starting with DCM finishing up with DCM/MeOH/NH₄OH (600/400/10). Obtained: 0.128 mg of 1-(2-{[3-(4-chlorophenoxy)benzyl]amino}-3-phenylpropyl)piperidin-4-ol ditrifluoroacetate, 53% yield, of at least 95% purity. Mass spectrum (ESI) *m*/*z* (MH)⁺ 451.2,453.2.

## Claims

1. A compound selected from the group consisting of: 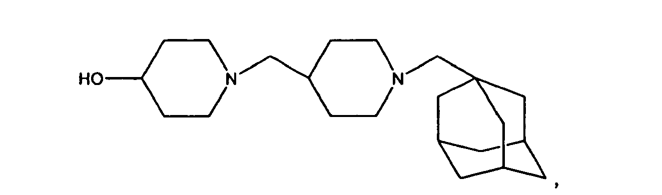 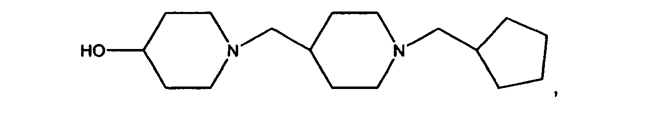 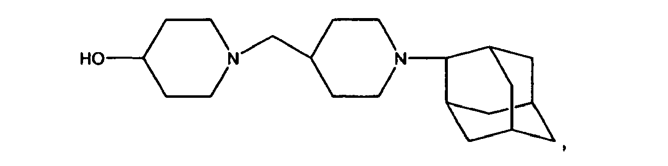 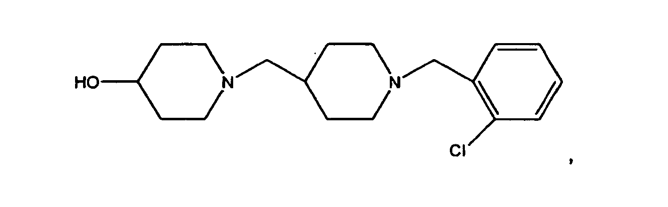 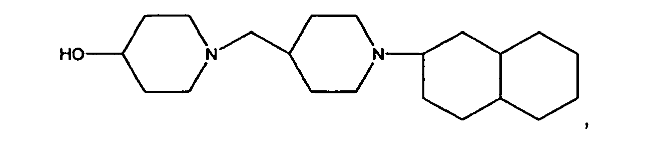 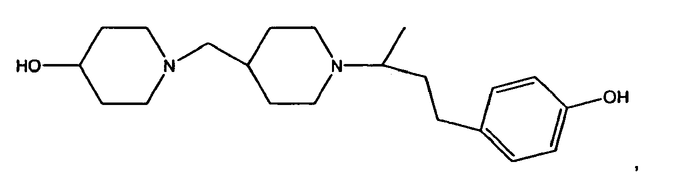 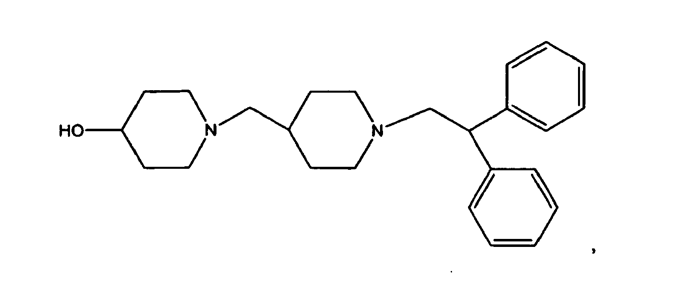 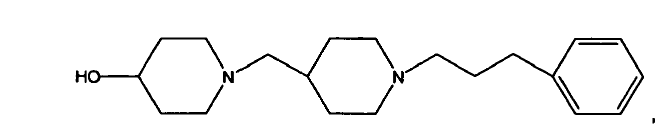 and

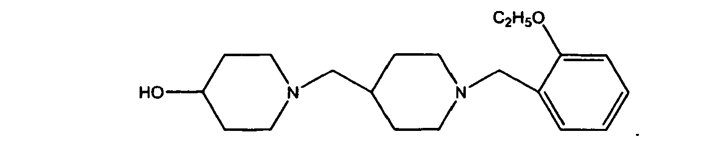

2. A pharmaceutical composition comprising a compound of Claim 1 and a pharmaceutically acceptable carrier.

3. The pharmaceutical composition of Claim 2, wherein the composition is for topical, oral, subcutaneous, intramuscular, intraocular, intraarterial, intravenous, or local administration using an implantable dosage unit.

4. The pharmaceutical composition of Claim 2, wherein the composition is in the form of an aerosol or a liquid.

5. The pharmaceutical composition of Claim 2, wherein the composition is in the form of a solid.

6. The pharmaceutical composition of Claim 2, wherein the composition is a tablet, a pill, a cream, a soap or an implantable dosage unit.

7. The pharmaceutical composition of Claim 6, wherein the composition comprises from 1 to 1000 mg of the compound.

8. A compound according to Claim 1 for use in the treatment of a mycobacterial infection in a human or other animal.

9. Use of a compound according to Claim 1 in the manufacture of a medicament for the treatment of a mycobacterial infection in a human or other animal.

10. The compound of Claim 8 or the use of Claim 9, wherein the mycobacterium is *M*. *tuberculosis, M. avium-intracellulare, M. kansarii, M. fortuitum, M. chelonae, M. leprae, M. africanum, M. bovis, M. microti, M. avium paratuberculosis, M. intracellulare, M. scrofulaceum, M. xenopi, M. marinum,* or *M*. *ulcerans.*

11. The compound of Claim 8 or the use of Claim 9, further comprising a pharmaceutically acceptable carrier.

12. The compound of Claim 8 or the use of Claim 9, wherein the mycobacterium is a drug resistant mycobacterial strain.

13. The compound of Claim 8 or the use of Claim 9, wherein the compound is administered topically, orally, subcutaneously, intramuscularly, intraocularly, intraarterially, intravenously, or locally using an implantable dosage unit.

14. The compound of Claim 8 or the use of Claim 9, wherein the compound is administered as a solid, liquid or aerosol.

15. The compound of Claim 8 or the use of Claim 9, wherein the compound is administered as a pill, a cream, a soap or an implantable dosage unit.

16. The compound of Claim 8, wherein the amount of the compound used is from 100 to 0.1 mg per kg of body weight, or from 50 to 0.2 mg per kg of body weight, or from 25 to 0.5 mg per kg of body weight, or is from 1 to 1000 mg.

## Patentansprüche

1. Verbindung, ausgewählt aus der Gruppe von: 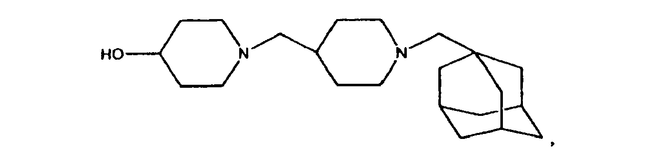 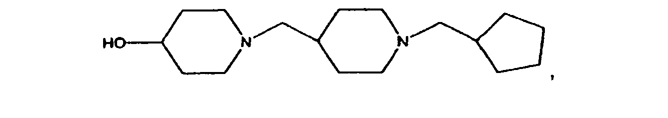 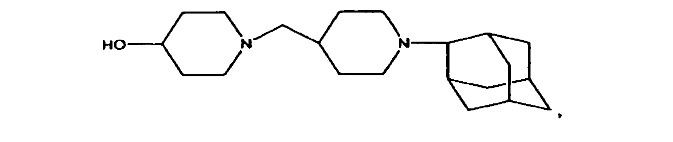 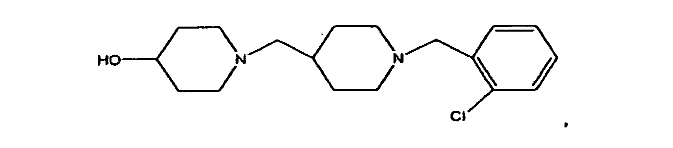 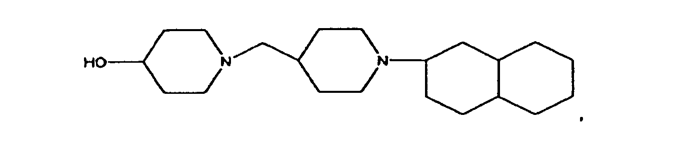 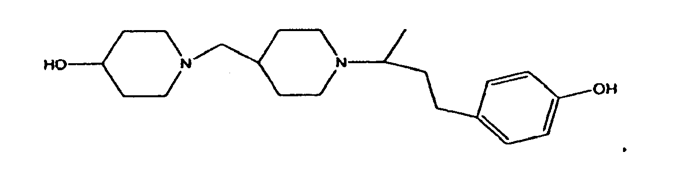 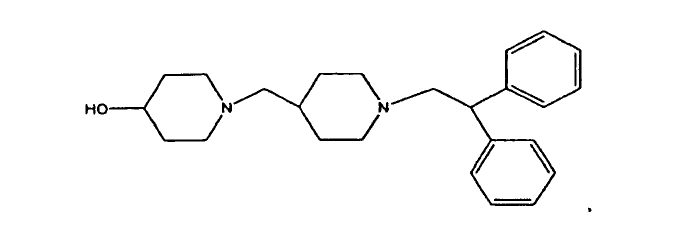 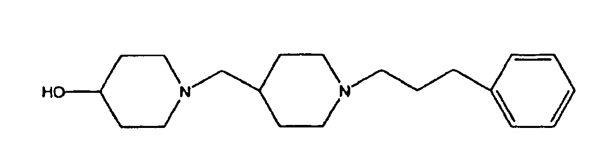 und

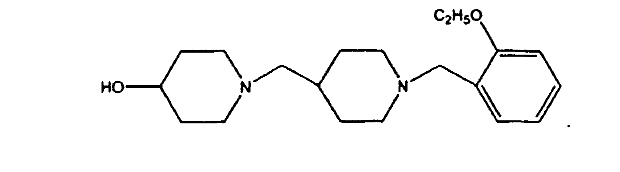

2. Pharmazeutische Zusammensetzung, die eine Verbindung nach Anspruch 1 und einen pharmazeutisch akzeptablen Träger umfasst.

3. Pharmazeutische Zusammensetzung nach Anspruch 2, wobei die Zusammensetzung zur topischen, oralen, subkutanen, intramuskulären, intraokulären, intraarteriellen, intravenösen oder lokalen Verabreichung unter Verwendung einer implantierbaren Dosierungseinheit dient.

4. Pharmazeutische Zusammensetzung nach Anspruch 2, wobei die Zusammensetzung in der Form eines Aerosols oder einer Flüssigkeit vorliegt.

5. Pharmazeutische Zusammensetzung nach Anspruch 2, wobei die Zusammensetzung in der Form eines Feststoffs vorliegt.

6. Pharmazeutische Zusammensetzung nach Anspruch 2, wobei die Zusammensetzung eine Tablette, eine Pille, eine Creme, eine Seife oder eine implantierbare Dosierungseinheit ist.

7. Pharmazeutische Zusammensetzung nach Anspruch 6, wobei die Zusammensetzung 1 bis 1000 mg der Verbindung umfasst.

8. Verbindung nach Anspruch 1 zur Verwendung bei der Behandlung einer mycobakteriellen Infektion bei einem Menschen oder einem anderen tierischen Lebewesen.

9. Verwendung einer Verbindung nach Anspruch 1 bei der Herstellung eines Medikaments zur Behandlung einer mycobakteriellen Infektion bei einem Menschen oder einem anderen tierischen Lebewesen.

10. Verbindung nach Anspruch 8 oder Verwendung nach Anspruch 9, wobei es sich bei dem Mycobacterium um *M. tuberculosis, M. avium-intracellulare, M. kansarii, M. fortuitum, M. chelonae, M. leprae, M. africanum, M. bovis, M. microti, M. avium paratuberculosis, M. intracellulare, M. scrofulaceum, M. xenopi, M. marinum* oder *M. ulcerans* handelt.

11. Verbindung nach Anspruch 8 oder Verwendung nach Anspruch 9, wobei ferner ein pharmazeutisch akzeptabler Träger umfasst ist.

12. Verbindung nach Anspruch 8 oder Verwendung nach Anspruch 9, wobei das Mycobacterium ein arzneistoffresistenter Mycobakterienstamm ist.

13. Verbindung nach Anspruch 8 oder Verwendung nach Anspruch 9, wobei die Verbindung topisch, oral, subcutan, intramuskulär, intraokulär, intraarteriell, intravenös oder lokal unter Verwendung einer implantierbaren Dosierungseinheit verabreicht wird.

14. Verbindung nach Anspruch 8 oder Verwendung nach Anspruch 9, wobei die Verbindung als Feststoff, Flüssigkeit oder Aerosol verabreicht wird.

15. Verbindung nach Anspruch 8 oder Verwendung nach Anspruch 9, wobei die Verbindung als Pille, Creme, Seife oder implantierbare Dosierungseinheit verabreicht wird.

16. Verbindung nach Anspruch 8, wobei die Menge der verwendeten Verbindung 100 bis 0,1 mg pro kg des Körpergewichts oder 50 bis 0,2 mg pro kg des Körpergewichts oder 25 bis 0,5 mg pro kg des Körpergewichts beträgt oder 1 bis 1000 mg beträgt.

## Revendications

1. Composé sélectionné dans le groupe constitué par : 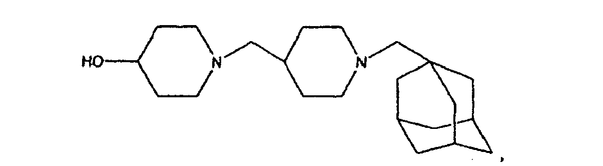 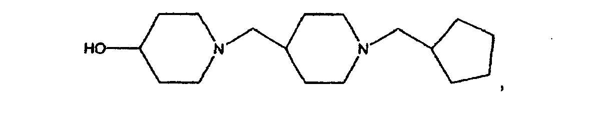 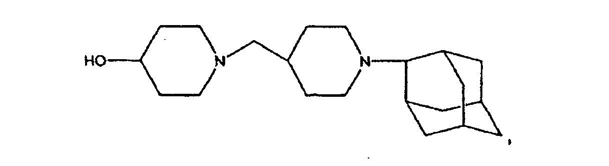 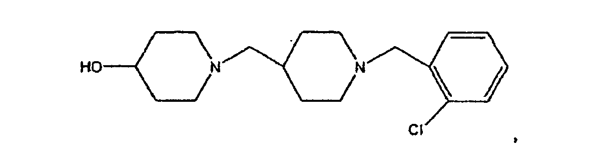 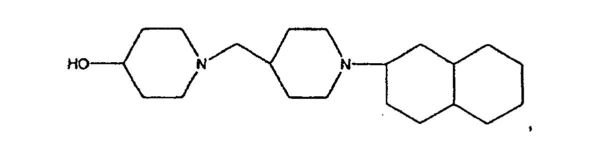 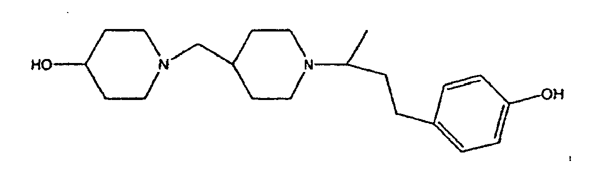 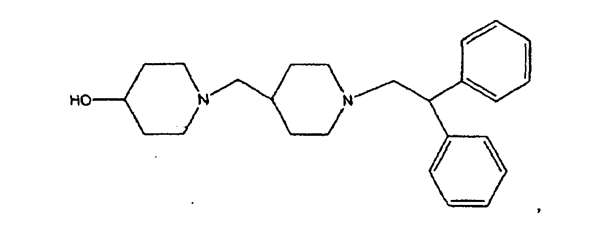 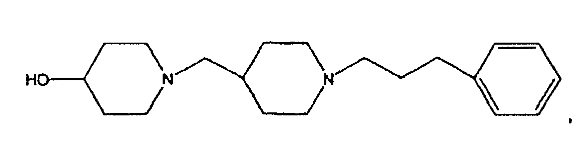 et

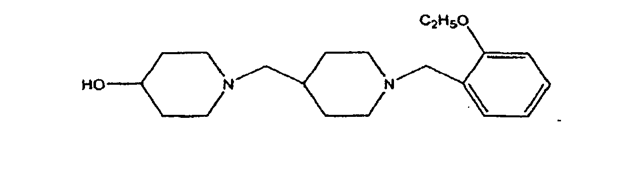

2. Composition pharmaceutique comprenant un composé de la revendication 1 et un vecteur pharmaceutiquement acceptable.

3. Composition pharmaceutique de la revendication 2, laquelle composition est destinée à une administration topique, orale, sous-cutanée, intramusculaire, intra-oculaire, intra-artérielle, intraveineuse ou locale à l'aide d'une unité de dosage implantable.

4. Composition pharmaceutique de la revendication 2, laquelle composition est sous la forme d'un aérosol ou d'un liquide.

5. Composition pharmaceutique de la revendication 2, laquelle composition est sous la forme d'un solide.

6. Composition pharmaceutique de la revendication 2, laquelle composition est un comprimé, une pilule, une crème, un savon ou une unité de dosage implantable.

7. Composition pharmaceutique de la revendication 6, laquelle composition comprend de 1 à 1 000 mg du composé.

8. Composé selon la revendication 1 pour utilisation dans le traitement d'une infection mycobactérienne chez un humain ou un autre animal.

9. Utilisation d'un composé selon la revendication 1 dans la fabrication d'un médicament pour le traitement d'une infection mycobactérienne chez un humain ou un autre animal.

10. Composé de la revendication 8 ou utilisation de la revendication 9, dans lequel/laquelle la mycobactérie est *M. tuberculosis, M. avium-intracellulare, M. kansarii, M. fortuitum, M. chelonae, M. leprae, M. africanum, M. bovis, M. microti, M. avium paratuberculosis, M. intracellulare, M. scrofulaceum, M. xenopi, M. marinum* ou *M. ulcerans.*

11. Composé de la revendication 8 ou utilisation de la revendication 9, comprenant en outre un véhicule pharmaceutiquement acceptable.

12. Composé de la revendication 8 ou utilisation de la revendication 9, dans lequel/laquelle la mycobactérie est une souche mycobactérienne résistante aux antibiotiques.

13. Composé de la revendication 8 ou utilisation de la revendication 9, dans lequel/laquelle le composé est administré par voie topique, orale, sous-cutanée, intramusculaire, intra-oculaire, intra-artérielle, intraveineuse ou locale à l'aide d'une unité de dosage implantable.

14. Composé de la revendication 8 ou utilisation de la revendication 9, dans lequel/laquelle le composé est administré sous forme de solide, de liquide ou d'aérosol.

15. Composé de la revendication 8 ou utilisation de la revendication 9, dans lequel/laquelle le composé est administré sous forme de pilule, de crème, de savon ou d'unité de dosage implantable.

16. Composé de la revendication 8, dans lequel la quantité utilisée va de 100 à 0,1 mg par kg de poids corporel, ou de 50 à 0,2 mg par kg de poids corporel, ou de 25 à 0,5 mg par kg de poids corporel, ou de 1 à 1 000 mg.
